# EUROPEAN PATENT SPECIFICATION

(11) **EP 0 888 326 B1**
(45) Date of publication and mention of the grant of the patent: **16.08.2006**
(21) Application number: 97907860.7
(22) Date of filing: 20.02.1997
(51) Int. Cl.: C07D 311/92, C07D 307/92, C07C 50/32, A61K 31/35, A61K 31/34, A61K 31/12

(54) **NOVEL ORTHO-NAPHTHOQUINONE DERIVATIVES, NOVEL SYNTHESIS THEREFOR, AND THEIR USE IN THE INHIBITION OF NEOPLASTIC CELL GROWTH**
NEUE ORTHO-NAPHTHOCHINONDERIVATE, NEUE SYNTHESEN ZU IHRER HERSTELLUNG UND IHRE ANWENDUNG IN DER INHIBIERUNG VON NEOPLASTISCHEM ZELLWACHSTUM
NOUVEAUX DERIVES D'ORTHO-NAPHTHOQUINONE, PROCEDE DE SYNTHESE CORRESPONDANT ET UTILISATION DE CES DERIVES POUR INHIBER LA CROISSANCE CELLULAIRE NEOPLASIQUE

(30) Priority: 20.02.1996 US 604131; 06.11.1996 US 744631
(43) Date of publication of application: 07.01.1999
(73) Proprietor: WISCONSIN ALUMNI RESEARCH FOUNDATION, Madison, Wisconsin 53707-7365 (US)
(72) Inventor: FRYDMAN, Benjamin, J., Madison, WI 53711 (US); WITIAK, Donald, T., Madison, WI 53711 (US); MARTON, Laurence, J., Madison, Wi 53711 (US); NEDER, Karen, Madison, WI 53713 (US); DOLAN, Eileen, M., Oak Park, IL 60304 (US); GEISER, Andrew, H., Madison, WI 53715 (US); SUN, Jerry, Shunneng, Madison, WI 53705 (US)
(74) Representative: Ellis-Jones, Patrick George Armine
(86) International application number: PCT/US1997/002955
(87) International publication number: WO 1997/031936

(56) References cited:
- WO-A-94/03163
- WO-A-94/11382
- WO-A-95/12588
- WO-A-96/33988
- WO-A-97/07797
- WO-A-97/08162
- R. CASSIS ET. AL.: "Studies on Quinones. VIII(I): The application of Michael adducts from 2-hydroxy-1,4-naphthoquinones for the preparation of dihydronaphthopyrandiones." JOURNAL OF HETEROCYCLIC CHEMISTRY, vol. 19, no. 2, March 1982 (1982-03), pages 381-4, XP000655161
- J. N. LOPES ET. AL.: "In vitro and in vivo evaluation of the toxicity of 1,4-naphthoquinone and 1,2-naphthoquinone derivatives against Trypanosoma Cruzi" ANNALS OF TROPICAL MEDICINE AND PARASITOLOGY, vol. 72, no. 6, December 1978 (1978-12), pages 523-31, XP000655159 LONDON, GB
- W. J. DUNN III: "Structure-activity analysed by pattern recognition. The Asymmetric case." JOURNAL OF MEDICINAL CHEMISTRY, vol. 23, no. 6, June 1980 (1980-06), pages 595-9, XP000655204 WASHINGTON DC, US
- F. J. BULLOCK ET. AL.: "Antiprotozoal Quinones. II. Synthesis of 4-amino-1,2-naphthoquinones and related compounds as potential antimalarials." JOURNAL OF MEDICINAL CHEMISTRY, vol. 13, no. 1, January 1970 (1970-01), pages 97-103, XP000655205 WASHINGTON DC, US
- K. KOBAYASHI ET. AL.: "Photoinduced molecular transformations. Part 127. A new [2+2] photoaddition of 2-amino-1,4-naphthoquinone with vinylarenes..." JOURNAL OF THE CHEMICAL SOCIETY, PERKIN TRANSACTIONS I., no. 1, January 1992 (1992-01), pages 115-21, XP000655153
- H. KAKISAWA ET. AL.: "Synthesis of Furonaphthoquinones." BULLETIN OF THE CHEMICAL SOCIETY OF JAPAN, vol. 43, no. 3, March 1970 (1970-03), pages 824-6, XP000655173 TOKYO, JP
- K. SCHAFFNER-SABBA ET. AL.: "Beta-Lapachone: synthesis of derivatives and activities in tumor models." JOURNAL OF MEDICINAL CHEMISTRY, vol. 27, no. 8, August 1984 (1984-08), pages 990-4, XP000651650 WASHINGTON DC, US
- R. B. GUPTA ET. AL.: "Direct oxidation of 1-naphthaldehydes to 2-substitued 1,4-naphthoquinones: application to the chemoselective syntheses of pyrano- and furano- 1,2-naphthoquinones." SYNLETT, no. 6, June 1990 (1990-06), pages 355-7, XP002034238 STUTTGART, DE
- CHEMICAL ABSTRACTS, vol. 93, no. 11, 15 September 1980 (1980-09-15) Columbus, Ohio, US; abstract no. 114285r, R. B. GUPTA ET. AL.: "Bromination with N-bromosuccinimide. Part III. Formation of 3'-bromo-beta-lapachone , dehydroiso-beta-lapachone and 4'-bromo-iso-beta-lapachone from lapachol." page 705; column 1; XP002034239 & INDIAN JOURNAL OF CHEMISTRY, SECT. B,, vol. 19B, no. 1, pages 17-19,
- A. V. PINTO ET. AL.: "Trypanocidal Activity of Synthetic Heterocyclic Derivatives of Active Quinones form Tabebuia sp." ARZNEIMITTEL FORSCHUNG, vol. 47, no. 1, 1997, pages 74-9, XP000999179
- CARDOSO, S.R.F. ET. AL.: "Synthesis of Some Naturally Occurring Naphthoquinones from Paratecoma and Tabebuia Species" ANNALEN ACADEMIE BRASIL CIENCIA, vol. 69, no. 1, 1997, pages 15-18, XP001000830

## Description

### FIELD OF THE INVENTION

This invention is directed to novel *o*-quinone and 4-substituted *o-*naphthoquinone derivatives which are structurally related to naturally-occurring lapachones and dunniones. The present invention is also drawn to a novel synthetic method to produce the lapachone derivatives, as well as the use of these compounds in the inhibition of neoplastic cell growth.

### DESCRIPTION OF THE PRIOR ART

The present invention is drawn toward novel bi- and tricyclic naphthoquinone derivatives, a synthetic method for making the derivatives, and use of the derivatives to inhibit neoplastic cell proliferation. The naphthoquinone derivatives of the present invention are related to the compounds known by their trivial names as β-lapachone (1) (7,8-dihydro-2,2-dimethyl-2H-naphthy(2,3-b)dihydropyran-7,8-dione) and dunnione (2) (2,3,3-trimethyl-2,3,4,5-tetrahydro-naphtho(2,3-b)dihydrofuran-6,7-dione).

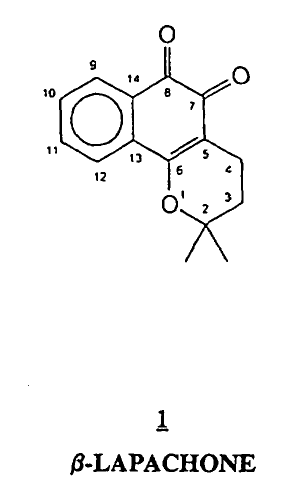

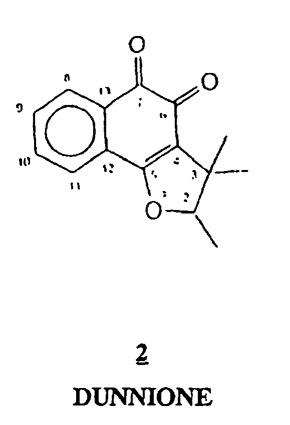

β-lapachone is a naturally occurring product which can be found in small amounts in the lapacho tree (*Tabebuia avellanedae*) of South America. β-Lapachone may also be readily synthesized from lapachol (3), an abundant quinone which is also found in the lapacho tree.

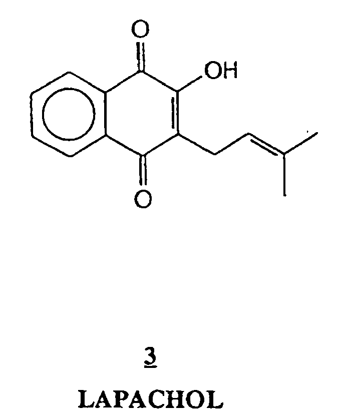

In similar fashion, dunnione (2) and its structural isomer α-dunnione (2a) (2,2,3-trimethyl-2,3,4,5-tetrahydro-naphtho(2,3-b)dihydro-furan-6,7-dione) can be isolated from the leaves of *Streptocarpus dunnii.*

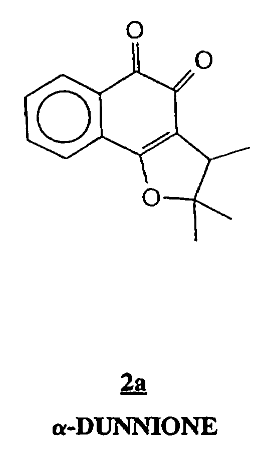

Early work on the synthesis of these related naphthodihydrofurandiones and naphthodihydropyrandiones began with Fieser's 1927 synthesis of lapachol (3) (Fieser, L.F. (1927), *J.A.C.S.,* **49:** 857.) The first known synthesis of dunniones (2 and 2a) and related naphthoquinones was performed by R.G. Cooke and co-workers at the University of Melbourne using a modification of Fieser's above-noted synthesis. (Cooke, R.G. et al. (1950), *Australian J. of Scient. Res.,* **3**:481-94.) In short, the Fieser method synthesizes β-lapachol (3) via alkylation of the silver salt of lawsone (*i.e*., 2-hydroxy-1,4-naphthoquinone) with dimethylallyl bromide in absolute ether. This synthetic route yields both the C-alkylated product (lapachol), as well as an O-alkylated by-product.

*Cooke et al.* modified this general procedure by beginning with the potassium salt of lawsone rather than the silver salt. The C-alkylated product (lapachol) is separated from the O-alkylated intermediate by acidification, which precipitates the lapachol from solution.

What is left behind is an O-alkylated lawsone derivative, namely, 2-(3',3'-dimethylalloxy)-1,4-naphthoquinone (4):

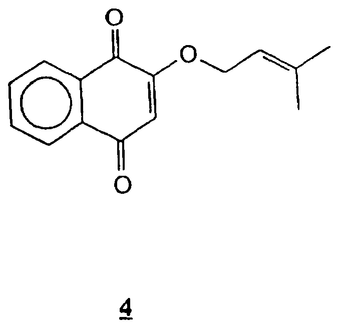

Compound (4) is then subjected to a Claisen rearrangement by refluxing in absolute ethanol to yield 2-hydroxy-3-(1',1'-dimethylallyl)-1,4-naphthoquinone(5):

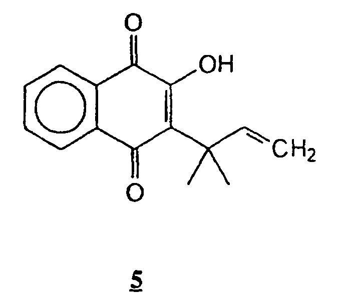

Under these mild conditions, *Cooke et al,* report that the conversion of (4) to (5) is practically quantitative. However, under more rigorous conditions, mixtures of the 1,2-dimethylallyl and 2,3-dimethylallyl isomers are also found.

Treatment of compound (5) with concentrated sulfuric acid yields a 2-ethyl-2-methyl derivative of dunnione (6):

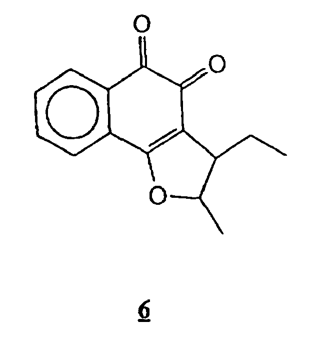

In an interesting aside, Professor Cooke appears to have maintained a life-long interest in the dunniones as evidenced by his 1980 paper "Crystal Structure of Dunnione *p*-Bromophenylhydrazone," which was published 30 years after the above-described reference. (*Aust. J. Chem.* (1980), **33**:442-5.)

A group of researchers led by Kenichiro Inoue has published several articles discussing the structure and biosynthetic pathways of various naphthoquinones isolated from *S. dunnii.* For instance, in a communication to the editor, *Inoue et al.* report the structure of several prenylated naphthoquinones from *S. dunnii.* In addition to dunnione, this reference also describes the isolation and characterization of 7-hydroxydunnione, 8-hydroxydunnione, and dehydrodunnione. This reference also describes the 1,4-naphthoquinone isomer of α-dunnione. This reference is limited solely to isolating the above-noted compounds from cell cultures of *S*. *dunnii.* A full-length paper describing the isolation and characterization of these naphthoquinones can be found in a 1983 publication of *Inoue et al.* (*Phytochemistry* (1983), **22**(3):737-741.)

In a follow-up paper (*Phytochemistry* (1984), **23**(2):313-318) *Inoue et al.* report a radioisotopic study to determine the biosynthetic pathways of several naphthoquinones and anthroquinones isolated from *S*. *dunnii. Inoue et al.* performed this study by inserting ¹³C- and ²H-labeled precursors into cell cultures of *S. dunnii.* The fate of the isotopically-labeled precursors was then tracked. *Inoue et al.* conclude that all of the dunnione naphthoquinone derivatives are biosynthesized via a common 4-(2'- carboxyphenol)-4-oxobutanoic acid precursor. This reference describes the formation of the deuterated 2-prenyl ether of lawsone by reacting lawsone with dimethylallyl bromide in the presence of potassium carbonate. Both deuterated lawsone and the deuterated 2-prenyl ether of lawsone were then administered to cell cultures of *S. dunnii* to determine the intermediacy of lawsone in the synthesis of dunnione. It must be noted, however, that this reference is silent regarding artificial methods for synthesizing dunnione and dunnione derivatives. Rather, *Inoue et al.* are limited to a discussion regarding possible biological pathways for the synthesis of naphthoquinones and anthraquinones in *S. dunnii.*

A German-language reference to *Rüedi and Eugster* describes the isolation of partially racemic (-)-dunnione from *Calceolaria integrifolia.* (*Rüedi and Eugster* (1977), *Hel. Chim. Acta,* **60**(3) 96:945-947.) According to the authors, this appears to be the first record of the occurrence of dunnione outside the family *Gesneriaceae.* In *Gesneriaceae,* dunnione is usually found as the dextrorotary enantiomer.

While dunniones, lapachones, and several derivatives thereof have been described in the prior art, no biological utility has been described in any of the above references, nor has any direct utility been described for any of the above-noted naphthofurandione or naphthopyrandione derivatives.

In the patent literature, *Adams et al.,* U.S. Patent No. 4,778,805, describe 4,7-benzofurandione derivatives which are useful as inhibitors of leukotriene synthesis. Because the benzofurandiones described by *Adams et al.* tend to inhibit mammalian leukotriene biosynthesis, they are described as useful therapeutic agents for treating allergic conditions, asthma, psoriasis, and other maladies which are biologically mediated by various leukotrienes.

*Petraitis et al.,* U.S. Patent No. 5,244,917, describe a large number of substituted naphthofurans which find use as anti-inflammatory agents.

1,2-naphthoquinones (also known as *ortho*-naphthoquinones, and referred to hereinbelow as *o*-naphthoquinones) have been widely investigated and find use in several different technological fields. For instance, German Patent Publication (*Offenlegungsschrift*) DE 3 926 747 A1 describes a large number of *o-*naphthoquinones having various substituents at the 4-position. These compounds are described as having fungicidal activity.

A 1974 report from the United States National Cancer Institute (Driscoll *et al.*) contains structure-activity data for more than 1,500 quinone derivatives using several *in vivo* mice models. The cancer types utilised in the *in vivo* mice models include L1210 (a leukemia), and W-256, CA-755, and S-180 (solid tumours). KB cancer cells were utilised for *in vitro* cytotoxicity testing. This reference contains a description of 4-ethoxy-1,2-napthoquinone (entry 1484) and notes its lack of *in vitro* cytotoxicity against KB cancer cells. The reported ED₅₀ is 8.0 µm/mL. As stated in the report itself, a compound is not considered cytotoxic unless it displays an ED₅₀ ≤ 4.0 in the *in vitro* KB cell model.

Mura *et al.,* U.S. Patent No. 4,879,243, describe the use of 4-methoxy-1,2-naphthoquinone and 4-methoxycarbonylmethoxy-1,2-naphthoquinone as electron transfer agents in an assay for the detection of cells in urine.

U.S. Patent No. 4,882,339, to Wasley, describes several 4-amino-substituted-1,2-dihydroxynaphthalene compounds which are reported to have inhibitory activity against 5-lipoxygenase. The 1,2-dihydroxynaphthalenes are synthesized via reduction of a corresponding 4-amino-1,2-naphthoquinone.

None of the references, taken alone or in any combination, are seen as describing the present disclosed invention.

K Schaffner-Sabba *et al* in J. Med. Chem. 1984, 27, 990 to 994 describe the synthesis of derivatives of β-lapachone and activity of those derivatives in tumour models. The synthesised β-lapachone derivatives are modified at position 2 of the pyran ring or are derivatised at positions 8 and 9 of the benzene ring.

### SUMMARY OF THE INVENTION

One aspect of the present invention is directed to a novel method to synthesize tricyclic o-naphthoquinones. The method is directed to synthesising compounds of Formula II: 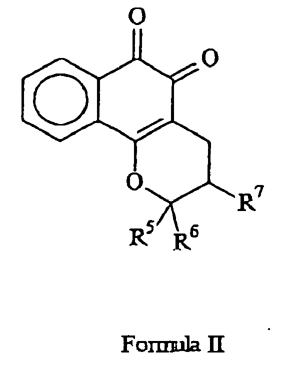 wherein R⁵ and R⁶ are each, independently, selected from the group consisting of H, C₁-C₆ alkyl, C₁-C₆ alkenyl, C₁-C₆ alkoxy, C₁-C₆ alkoxycarbonyl, -(CH₂)ₙ-aryl, -(CH₂)ₙ-heteroaryl, -(CH₂)ₙ-heterocycle, and -(CH₂)ₙ-phenyl; and R⁷ is OH, C₁-C₆ alkyl, C₁-C₆ alkenyl, C₁-C₆ alkoxy, C₁-C₆ alkoxycarbonyl, C₁-C₆ acyloxy, amino-C₂₋C₆-acyloxy, malonyloxy, malonyl, β-alanyloxy, -(CH₂)ₙ-amino, -(CH₂)ₙ-aryl, -(CH₂)ₙ₋heteroaryl, -(CH₂)ₙ-heterocycle, or (CH₂)ₙ-phenyl, wherein n is an integer of from 0 to 10.

The method includes the steps of alkylating a Group IA metal salt of lawsone with an allyl halide of the formula: 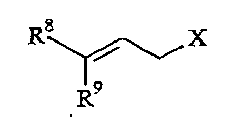 in the presence of M-I; wherein R⁸ and R⁹ are each, independently, selected from the group consisting ofH, C₁-C₆ alkyl, C₁-C₆ alkenyl, C₁-C₆ alkoxy, C₁-C₆ alkoxycarbonyl, -(CH₂)ₙ-aryl, -(CH₂)ₙ-heteroaryl, -(CH₂)ₙ-heterocycle, and -(CH₂)ₙ₋phenyl; and X is a halide, and M is a group IA metal; which yields a mixture of C-alkylated and O-alkylated lawsone derivatives.

The mixture of C-alkylated and O-alkylated lawsone derivatives is then cyclized to yield a tricyclic *ortho*-naphthoquinone.

The mixture of C-alkylated and O-alkylated lawsone derivatives is then separated from one another to yield a first portion of C-alkylated derivatives and a portion of O-alkylated derivatives. The portion of O-alkylated lawsone derivatives is then rearranged to yield a second portion of C-alkylated lawsone derivatives. The first and second portions of C-alkylated lawsone derivatives are cyclized to yield a tricyclic *ortho*-naphthoquinone.

Using the synthetic method described above, several novel compounds have been synthesized. The invention provides compounds selected from the group consisting of Formula I or II: 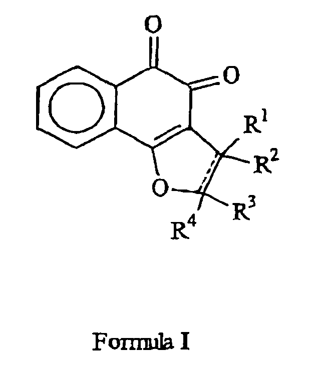 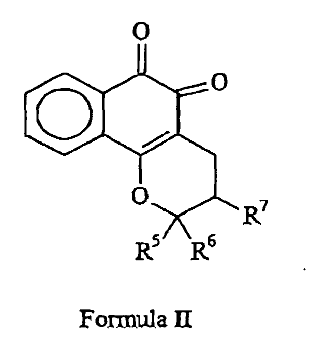 wherein
R¹ to R⁴ are each, independently, chosen from C₁-C₆ alkyl, C₁-C₆ alkenyl, C₁₋C₆ alkoxycarbonyl, -(CH₂)ₙ-aryl, -(CH₂)ₙ-heteroaryl, and -(CH₂)ₙ-phenyl, or R¹ and R² combined are a single substituent chosen from the above group and R³ and R⁴ combined are a single substituent chosen from the above group, in which case --- is a double bond;
R⁵ and R⁶ are each, independently, chosen from H, C₁-C₆ alkyl, C₁-C₆ alkenyl, C₁-C₆ alkoxy, C₁-C₆ alkoxycarbonyl, -(CH₂)ₙ-aryl, -(CH₂)ₙ-heteroaryl, and -(CH₂)ₙ-phenyl;
R⁷ is chosen from -(CH₂)ₙ-amino, β-alanyloxy, malonyl, malonyloxy, -(CH₂)ₙ-heteroaryl, and -(CH₂)ₙ-phenyl; and
n is from 0 to 10, and salts thereof, except that the compound is not 3-(2'-aminoethyl)-β-lapachone.

Novel compounds of Formula III are also included within the invention: 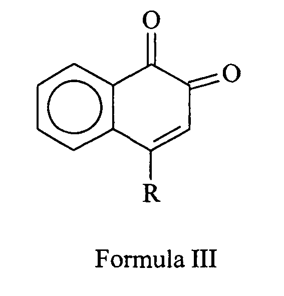 wherein R is selected from the group consisting of cyclohexyl-C₁-C₈ alkyloxy, C₅-C₈ alkenyloxy, and C₃-C₈ linear or branched alkylthio; and
Specifically included within the compounds of the present invention are 3-allyl-β-lapachone, 3-(β-alanyloxy)-β-lapachone, 3-malonyl-β-lapachone, 3-NOR-dunnione, 3,3-DINOR-dunnione, 3,3-DINOR-2,3-dehydrodunnione, 4-(n-pentylthio)-1,2-napthoquinone,4-(3-methyl-butyloxy)-1,2-napthoquinone,4-cyclohexylmethyloxy-1,2-napthoquinone, 4-(2-pentenyloxy)-1,2-napthoquinone, and 4-(n-heptyloxy)-1,2-napthoquinone.

A distinct advantage of the present synthetic method is that it allows for the synthesis of novel *o*-naphthoquinone derivatives, including lapachone and dunnione derivatives, using lawsone as a starting material. Lawsone is a commodity chemical which can be readily purchased in large quantities. This is a vast improvement over isolation of these products from natural sources or synthesis from naturally-occurring precursors.

The presently described synthetic method is also remarkably efficient and clean. The overall synthesis yields dunnione, lapachone, and other tricyclic o-naphthoquinone derivatives in heretofore unattainable yields and purity.

The synthesis is also quite easy, thereby avoiding the cumbersome manipulations required in prior art synthetic methods.

Of equal importance, the biological activity of the compounds described herein is remarkable in that the compounds are potent inhibitors of neoplastic cell growth and proliferation. The compounds described herein find use as chemotherapeutic agents in the treatment of a wide range of neoplasms, including cancers of the prostate, breast, colon, and lung. The compounds exhibit their anti-proliferative effects in heretofore unknown, minute concentrations.

In light of the newly-discovered biological activity of these compounds, another aspect of the present invention is drawn to a method of inhibiting growth of cancer cells by contacting the cells with one or more compounds described herein.

More specifically, this aspect of the invention is drawn to a composition for use in the treatment of cancer in mammals characterized in that it includes an effective cancer cell-growth inhibiting amount of a compound of Formula I, II, or III: 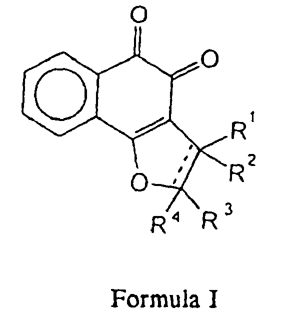 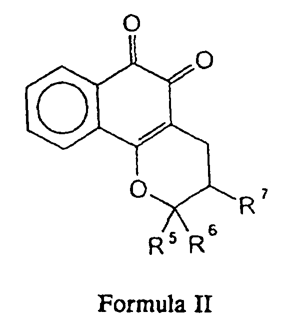 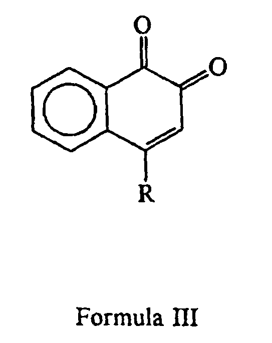 wherein R is selected from the group consisting of C₃-C₈ linear or branched alkyloxy, C₃-C₈ linear or branched alkenyloxy, C₃-C₈ linear or branched amino, C₃-C₈ alkylthio, and C₁-C₈ linear or branched substituted alkyloxy, alkenyloxy, amino, and alkylthio, substituted with one or more substituents selected from the group consisting of cycloalkyl, cycloalkenyl, cycloaryl, benzyl, and amino, and pharmaceutically-suitable salts thereof; and
wherein R¹ to R⁴ are each, independently, chosen from H, C₁-C₆ alkyl, C₁-C₆ alkenyl, C₁-C₆ alkoxy, C₁-C₆alkoxycarbonyl, -(CH₂)ₙ-aryl, -(CH₂)ₙ-heteroaryl, and -(CH₂-)ₙ-phenyl, provided that at least one of R¹ to R⁴ is not hydrogen; or R¹ and R² combined are a single substituent chosen from the above group and R³ and R⁴ combined are a single substituent chosen from the above group, in which case --- is a double bond, and further provided that the combined R¹/R² and R³/R⁴ substituents are not simultaneously hydrogen;
R⁵ and R⁶ are each, independently, chosen from H, C₁-C₆ alkyl, C₁-C₆ alkenyl, C₁-C₆ alkoxy, C₁-C₆ alkoxycarbonyl, -(CH₂)ₙ-aryl, -(CH₂)ₙ-heteroaryl, and -(CH₂)ₙ-phenyl;
R⁷ is chosen from C₁-C₆ acyloxy, -(CH₂)ₙ-amino, β-alanyloxy, malonyl, malonyloxy, -(CH₂)ₙ-aryl, -(CH₂)ₙ-heteroaryl, and -(CH₂)ₙ-phenyl; and
n is from 0 to 10, and salts thereof.

The composition may further include a pharmaceutically acceptable liquid or solid carrier.

More specifically, the composition includes one or more compounds selected from the group consisting of β-lapachone, 3-hydroxy-β-lapachone, 3-allyl-β-lapachone, 3-(*O*-acetyl)-β-lapachone, 3-(β-alanyloxy)-β-lapachone, 3-malonyl-β-lapachone, dunnione, α-dunnione, 3-NOR-dunnione, 3,3-DINOR-dunnione, 3.3-DINOR-2,3-dehydrodunnione, 4-pentyloxy-1,2-naphthoquinone; 4-(2-methyl-propyloxy)-1,2-naphthoquinone; 4-(3-methyl-butyloxy)-1,2-napbthoquinone; 4-(isopropyloxy)-1,2-naphthoquinone; 4-(2-butenyloxy)-1,2-naphthoquinone; 4-benzyloxy-1,2-naphthoquinone; 4-cyclohexylmethyloxy-1,2-naphthoquinone; 4-(2-pentenyloxy)-1,2-naphthoquinone; 4-(n-heptyloxy) 1,2-naphthoquinone; 4-(n-pentylthio)-1,2-naphthoquinone; 4-(2-N,N-dimethylamino-ethylamino)-1,2-naphthoquinone, and pharmaceutically acceptable salts thereof, and combinations thereof.

The composition noted above may be used in the treatment of multiple-drug resistant cancer, adriamycin-resistant cancer, teniposide-resistant cancer, and vinblastine-resistant cancer.

The method includes administering to a human cancer patient in need thereof an amount of one or more of the above-described compounds which is effective to inhibit the growth of the cancer cell.

The present method for the inhibition of neoplastic cell growth has both *in vivo* and *in vitro* applications. *In vivo,* the method encompasses the therapeutic treatment of neoplastic growths in mammals, including humans. The treatment includes administering an effective cancer cell growth-inhibiting amount of a compound as described above to a person or animal in need thereof. *In vitro,* the method for neoplastic cell growth inhibition is effective for inhibiting the proliferation of a large number of different human cancer cell lines, including breast cancer, lung cancer, colon cancer, and prostate cancer.

The invention is also drawn to a pharmaceutical unit dosage form for the treatment of neoplastic cell growth characterized in that the dosage form contains one or more of the compounds described above, optionally in combination with a pharmaceutically suitable carrier.

The invention is further drawn to novel compounds of Formula I, II, and III as described above.

The biological activity of the compounds described herein is remarkable in that the compounds are potent inhibitors of neoplastic cell growth and proliferation at extremely low concentrations. Even more remarkable, several of the compounds display activity against adriamycin-resistant (ADR-resistant) neoplastic cell lines, as well as multi-drug resistant (MDR) neoplastic cell lines. The compounds described herein find use as chemotherapeutic agents in the treatment of a wide range of neoplasms, including cancers of the prostate, breast, colon, brain, and lung. The compounds exhibit their anti-proliferative effects in heretofore unknown, minute concentrations.

In light of the above discussion, a principal aim of the present invention is to provide novel compounds and pharmaceutical compositions which inhibit the growth of cancer cells both *in vitro* and *in vivo* at very low dosages.

It is further an aim of the present invention to provide novel synthetic methods for making the compounds described herein. Specifically, it is an aim of the present invention to provide a novel synthetic methodology for the manufacture of *o-*naphthoquinone derivatives, including tricyclic *o*-naphthofurandione and *o-*naphthopyrandione derivatives.

Further aims, objects, and advantages of the presently described synthetic methods and products will become clear upon a complete reading of the following Detailed Description, drawings, and attached claims.

### BRIEF DESCRIPTION OF THE DRAWINGS

**Fig. 1** is a semi-log graph depicting the fraction of cell survival in the presence of β-lapachone and 3-hydroxy-β-lapachone of colon cancer cells HT29.
**Fig. 2** is a semi-log graph depicting the fraction of cell survival in the presence of β-lapachone and 3-hydroxy-β-lapachone of colon cancer cells BE.
**Fig. 3** is a semi-log graph depicting the fraction of cell survival in the presence of β-lapachone and 3-hydroxy-β-lapachone of lung cancer cells A549.
**Fig. 4** is a semi-log graph depicting the fraction of cell survival of A549 lung cancer cells in the presence of β-lapachone and 2-methyl-2,3,4,5-tetrahydro-naphtho(2,3-b)dihydrofuran-6,7-dione(also called 3,3-DINOR-dunnione), designated in the graph as "Drug A."
**Fig. 5** is a semi-log graph depicting the fraction of cell survival of A549 lung cancer cells in the presence of Drug C (dunnione, i.e., 2,3,3-trimethyl-2,3,4,5-dihydro-naphtho(2,3-b)dihydrofuran-6,7-dione) and Drug B (2,3-dimethyl-2,3,4,5-dihydro-naphtho(2,3-b)dihydrofuran-6,7-dione, also called 3-NOR-dunnione).
**Fig. 6** is a semi-log graph depicting the fraction of cell survival of breast cancer cells MCF7 in the presence of β-lapachone and 3-hydroxy-β-lapachone.
**Fig. 7** is a semi-log graph depicting the fraction of cell survival of breast cancer cells MCF7 in the presence of β-lapachone and Drug A (2-methyl-2,3,4,5-tetrahydro-naphtho(2,3-b)dihydrofuran-6,7-dione, also called 3,3-DINOR-dunnione).
**Fig. 8** is a semi-log graph depicting the fraction of cell survival of breast cancer cells MCF7 in the presence of Drug C (dunnione) and Drug B (2,3-dimethyl-2,3,4,5-dihydro-naphtho(2,3-b)dihydrofuran-6,7-dione, also called 3-NOR-dunnione).
**Figs. 9A and 9B** combined depict an electrophoresis plate run illustrating the ability of several of the compounds described herein to induce Topoisomerase II-mediated cleavage of DNA. The far left-hand lane of Fig. 9A is a lane containing DNA alone and the adjacent lane contains DNA and Topo II. The groups of lanes labeled 1-9 in Figs. 9A and 9B depict varying concentrations of different compounds described herein in combination with DNA and Topo II.
**Fig. 10** is a graph showing the *in vitro* effect of increasing concentrations of 4-pentyloxy-1,2-napthoquinone on the survival of cultured MCF7 (ATCC HTB-22) breast cancer cells (●) and adriamycin-resistant MCF7 breast cancer cells (MCF7ADR) (○).
**Fig. 11** is a graph showing the *in vitro* effect of increasing concentrations of 4-pentyloxy-1,2-napthoquinone on the survival of cultured MCF7 breast cancer cells (●), adriamycin-resistant MCF7 breast cancer cells (MCF7ADR) (■), and MCF7 breast cancer cells containing a high level of cDNA-expressed glutathione peroxidase (MCF7GPX) (○).
**Fig. 12** is a graph showing the *in vitro* effect of increasing concentrations of 4-pentyloxy-1,2-napthoquinone on the survival of cultured CEM leukemia cells (ATCC CCL-119) (●), teniposide-resistant CEM leukemia cells (CEM-VM-1) (■), and merbarone-resistant leukemia cells (CEM/M70-B1) (○).
**Fig. 13** is a graph showing the *in vitro* effect of increasing concentrations of 4-(2-methyl-propyloxy)-1,2-napthoquinone on the survival of cultured MCF7 breast cancer cells (●) and adriamycin-resistant MCF7 breast cancer cells (MCF7ADR) (○).
**Fig. 14** is a graph showing the *in vitro* effect of increasing concentrations of 4-(3-methyl-butyloxy)-1,2-napthoquinone on the survival of cultured MCF7 breast cancer cells (●) and adriamycin-resistant MCF7 breast cancer cells (MCF7ADR) (○).
**Fig. 15** is a graph showing the *in vitro* effect of increasing concentrations of 4-(isopropyloxy)-1,2-napthoquinone on the survival of cultured MCF7 breast cancer cells (●) and adriamycin-resistant MCF7 breast cancer cells (MCF7ADR) (○).
**Fig. 16** is a graph showing the *in vitro* effect of increasing concentrations of 4-(2-butenyloxy)-1,2-napthoquinone on the survival of cultured MCF7 breast cancer cells (●) and adriamycin-resistant MCF7 breast cancer cells (MCF7ADR) (○).
**Fig. 17** is a graph showing the *in vitro* effect of increasing concentrations of 4-benzyloxy-1,2-napthoquinone on the survival of cultured MCF7 breast cancer cells (●) and adriamycin-resistant MCF7 breast cancer cells (MCF7ADR) (○).
**Fig. 18** is a graph showing the *in vitro* effect of increasing concentrations of 4-cyclohexylmethyloxy-1,2-napthoquinone on the survival of cultured MCF7 breast cancer cells (●) and adriamycin-resistant MCF7 breast cancer cells (MCF7ADR) (○).
**Fig. 19** is a graph showing the *in vitro* effect of increasing concentrations of 4-(2-pentenyloxy)-1,2-napthoquinone on the survival of cultured MCF7 breast cancer cells (●) and adriamycin-resistant MCF7 breast cancer cells (MCF7ADR) (○).
**Fig. 20** is a graph showing the *in vitro* effect of increasing concentrations of 4-(n-heptyloxy)-1,2-napthoquinone on the survival of cultured MCF7 breast cancer cells (●) and adriamycin-resistant MCF7 breast cancer cells (MCF7ADR) (○).
**Fig. 21** is a graph showing the *in vitro* effect of increasing concentrations of 4-(n-pentylthio)-1,2-napthoquinone on the survival of cultured MCF7 breast cancer cells (●) and adriamycin-resistant MCF7 breast cancer cells (MCF7ADR) (○).
**Fig. 22** is a graph showing the *in vitro* effect of increasing concentrations of 4-(2-N,N-dimethylamino-ethylamino)-1,2-napthoquinone on the survival of cultured MCF7 breast cancer cells (●) and adriamycin-resistant MCF7 breast cancer cells (MCF7ADR) (○).
**Fig. 23** is a graph of the results of the Comparative Example, showing the minimal *in vitro* cytotoxicity of 4-ethoxy-1,2-naphthoquinone against cultured human lung cancer cells A549 (ATCC CCL-185) (●) and cultured human breast cancer cells MCF7 (■).

### DETAILED DESCRIPTION OF THE INVENTION

The compounds described herein may be prepared using the reactions, techniques, and general synthetic procedures described herein below. Each of the references cited below are hereby incorporated herein by reference. The various reactions may be performed in various solvents which are appropriate to the reagents and materials employed and which are suitable for the transformation being effected. It is understood by those skilled in the art of organic synthesis that the functionality present on portions of a given molecule must be compatible with the reagents and reaction conditions proposed.

The synthetic method described herein generally and preferably uses lawsone (2-hydroxy-1,4-naphthoquinone) as a starting reagent. Lawsone is a commodity chemical which can be purchased in kilogram quantities from several commercial suppliers. (For instance, the Aldrich Chemical Co., Inc., Milwaukee, Wisconsin.)

One aspect of the present invention is drawn to a preparative-scale alkylation of a Group IA metal salt of lawsone (2-hydroxy-1,4-naphthoquinone) with allyl halides to yield tricyclic *ortho*-naphthoquinones. As described in full, below, the resultant tricyclic *o*-naphthoquinones are potent inhibitors of neoplastic cell proliferation and growth. Consequently, the compounds described herein are useful in the therapeutic treatment of cancerous tumors and other neoplasms.

### Synthesis of Tricyclic o-Naphthoquinones

Alkylation of a Group IA metal salt of lawsone (*i.e.*, lithium lawsone, sodium lawsone, potassium lawsone, *etc*.) with an allyl halide yields a mixture of C-alkylated products and O-alkylated products. The present synthetic method utilizes these two intermediate products to synthesize tricylic *o*-naphthoquinone products such as β-lapachone, dunnione, α-dunnione, and related derivatives. The present inventors have discovered that these compounds are potent inhibitors of neoplastic cell growth and proliferation.

Reaction I illustrates the initial synthesis of the lawsone salt, followed by reaction with an allyl halide. For clarity and brevity, the remainder of the Detailed Description shall refer to the preferred embodiment of the synthesis, wherein the lawsone salt is a lithium salt and the allyl halide is an allyl bromide. This is for sake of clarity only. The presently described synthetic method functions with equal success using other Group IA metal salts of lawsone and other allyl halides, such as allyl chlorides.

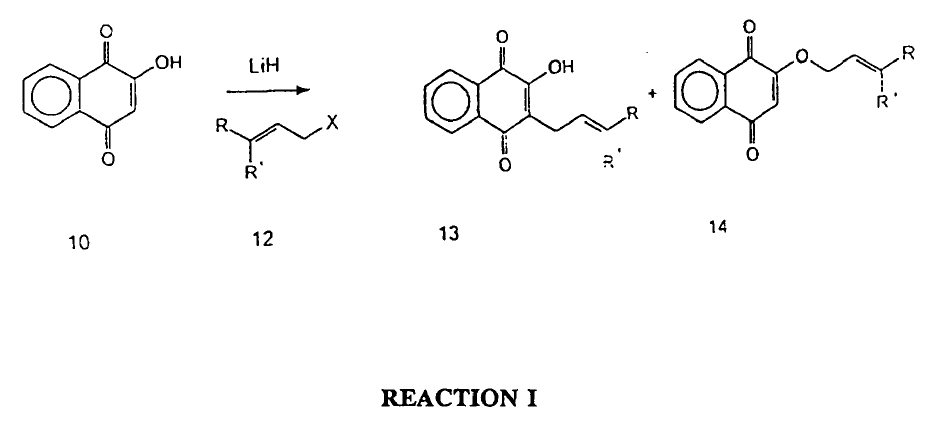

With reference to Reaction I, preferably, a lithium salt of lawsone is prepared by dissolving lawsone (10) in a suitable solvent, preferably dimethylsulfoxide (DMSO), and then adding lithium hydride, LiH. A novel and preferred protocol is to cool the lawsone solution to -78°C prior to the addition of the LiH, and then add the LiH to the solidified reaction mixture. The solution is then slowly warmed to room temperature. As the solution warms, the LiH is slowly dissolved into the reaction mixture, allowing for easy control of the evolution of hydrogen. The controlled evolution of hydrogen also eliminates the need to purge oxygen from the reaction solution by bubbling with a non-reactive gas. This makes generation of the lithium salt and the subsequent alkylation less cumbersome than prior art methods.

Alkylation proceeds by the addition of an allyl halide, preferably an allyl bromide (12) and a Group IA metal iodide, M-I, wherein M is a Group IA metal (preferably lithium), to the reaction mixture. Addition of the metal iodide serves to transform the allyl bromide *in situ* into an allyl iodide, which then reacts with the lawsone salt.

This *in situ* generation of an allyl iodide is another novel feature of the present invention. Allyl iodides are much more reactive than allyl bromides or allyl chlorides and are therefore desirable for use in alkylations. However, allyl iodides are also unstable and have a short shelf-life. Therefore, rather than working directly with the unstable allyl iodide, the allyl iodide is generated *in situ,* where it reacts with the lawsone salt. In addition, the high reactivity of the allyl iodide so formed increases the overall yield of the synthesis.

As shown Reaction I, R and R' are, independently, H, C₁-C₆ alkyl, C₁-C₆ alkenyl, C₁-C₆ alkoxy, C₁-C₆ alkoxycarbonyl, -(CH₂)ₙ-aryl, -(CH₂)ₙ-heteroaryl,-(CH₂)ₙ-heterocycle, or -(CH₂)ₙ-phenyl, or R¹ and R² combined are H, and R³ and R⁴ combined are H, in which case --- is a double bond: and R⁷ is H, C₁-C₆ alkyl, C₁-C₆ alkenyl, C₁-C₆ alkoxy, C₁-C₆ alkoxycarbonyl, -(CH₂)ₙ-aryl, -(CH₂)ₙ-heteroaryl,-(CH₂)ₙ-heterocycle, or -(CH₂)ₙ-phenyl, wherein n is an integer of from 0 to 10.

Reaction I yields a mixture of C-alkylated lawsone derivatives (13), O-alkylated derivatives (14), and unreacted lawsone (10).

The O-alkylated derivatives (14) precipitate from solution and can be separated by filtration, centrifugation, or any other suitable means for separating solids from a reaction solution. The unreacted lawsone and the C-alkylated derivatives (13) may then be isolated based upon differences in their respective acidities by any number of well known means.

Illustratively, the reaction mixture containing lawsone and C-alkylated derivatives (13) is acidified and extracted into ethyl acetate. The lawsone is then re-extracted into a sodium bicarbonate solution (~ 5%) and recovered by acidification. The less basic C-alkylated derivatives are recovered from the organic solvent by extraction with sodium hydroxide (~ 2N) and recovered by acidification.

Overall yield is approximately 40% for the C-alkyl derivatives and approximately 30% for the O-alkyl derivatives.

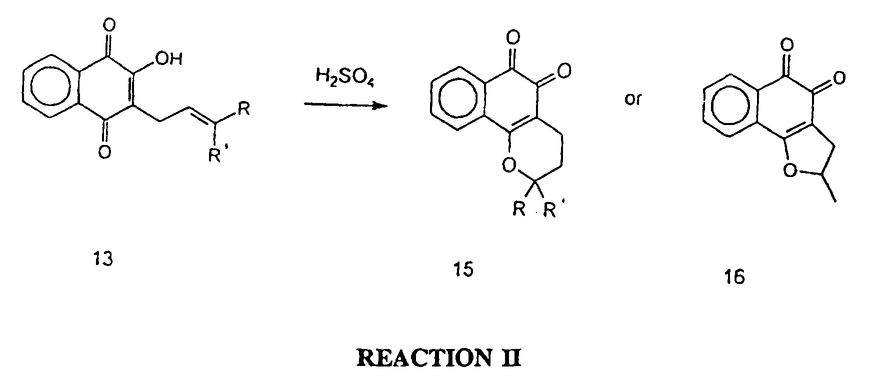

As shown in Reaction II, the C-alkylated naphthoquinone derivatives (13) can be converted into tricyclic *o*-naphthoquinone products *via* cyclization by treatment with concentrated sulfuric acid using well established procedures. (See, for instance, S.C. Hooker (1892), *J. Chem. Soc.,* **61**:611.) When R and/or R' are substituents other than hydrogen, the six-membered dihydro-naphthopyrandione derivatives (15) are formed. Presumably, this arises due to stabilization of the carbocation of the secondary or tertiary carbon center. If R and R' are both hydrogen, five-membered dihydro-naphthodihydrofurandiones (16) are obtained.

Reaction III shows a Claisen rearrangement of the O-alkylated derivatives (14) to yield a rearranged intermediate (17). As depicted, the rearrangement is accomplished by refluxing in toluene (although any suitable solvent will suffice). The Claisen rearrangement is well known to synthetic organic chemists. See, for instance, R.G. Cooke, *Australian J. Sci. Res.,* above. In the same fashion as Reaction II, the rearranged product (17) of Reaction III may be cyclized by treatment with concentrated sulfuric acid.

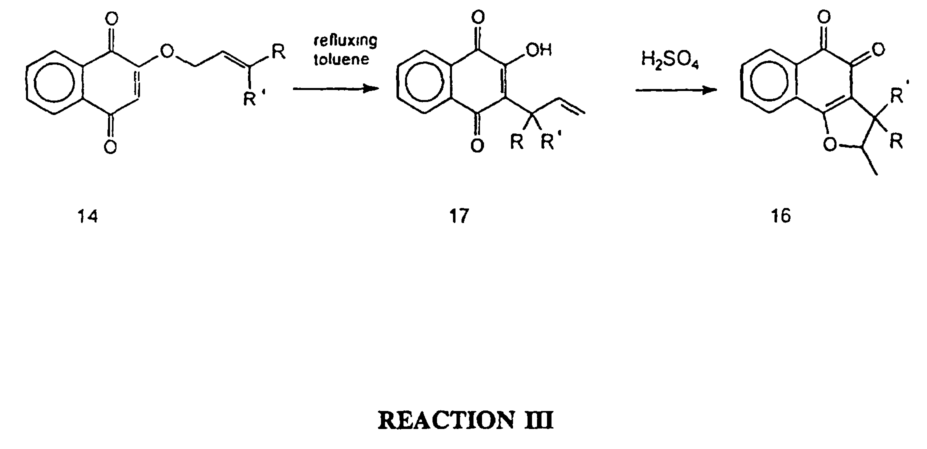

Additional derivatives of the above-described tricyclic *o*-naphthoquinone compounds can also be synthesized by modifying the synthetic pathway used to cyclize the third fused ring. An illustrative example, depicting the synthesis of 3-hydroxy-β-lapachone (19) is shown in Reaction IV, below: 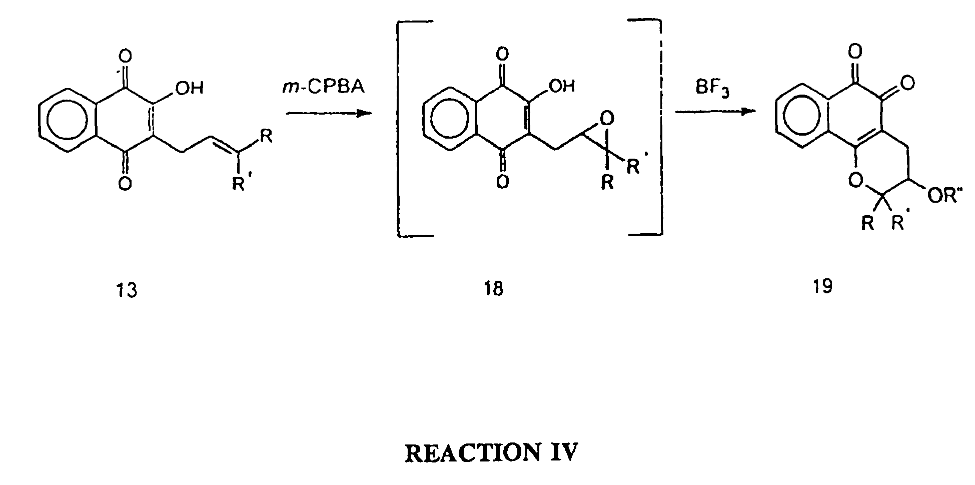 Here, an allyl derivative of lawsone, depicted in Reaction IV as lapachol (13) (wherein R and R' are methyl), is treated with *m*-chloroperoxybenzoic acid to afford the epoxide (18). Preferably, the epoxide is not isolated. Rather, the epoxide is directly transformed into the tricyclic *o*-naphthoquinone derivative (19) by treatment with boron trifluoride. The overall yield of product (19) is approximately 50%.

The reaction illustrated in Reaction IV is a distinct improvement over prior art ring-closure transformations leading from (13) to (19) because it is far less cumbersome. The intermediate need not be isolated and the yield is quite high.

Still other tricyclic *o*-naphthoquinone derivatives can be synthesized *via* the reaction depicted in Reaction V. Here, a fully aromatic derivative, 3,3-DINOR-2,3-dehydrodunnione, is synthesized. 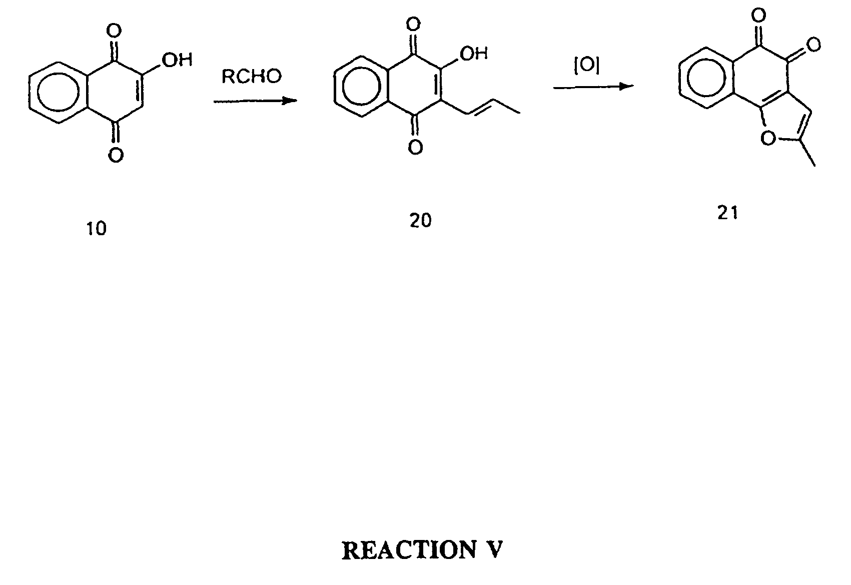 Here, lawsone is reacted with an aldehyde, such as propionaldehyde to yield a vinyl-*p*-quinone (20). The aldehyde may be selected from a wide range of suitable aldehydes and may contain additional functionalities. Suitable aldehydes include C₁₋C₆ linear or branched aliphatic aldehydes, as well as C₁-C₆ dicarbonyl, cylic, heterocyclic, aromatic, and heteroaromatic aldehydes. Cyclization of (20) under acid catalysis and mild oxidation yields the fully-aromatic dunnione derivative (21). Illustratively, the ring closure in Reaction V can be accomplished by treatment of (20) with mercuric acetate (Hg(CH₃CO₂)₂, a mild oxidant) in the presence of acetic acid.

The 3-hydroxy-naphtho(2,3-b)dihydropyran-7,8-dionederivatives(19)(wherein R" is hydrogen), shown in Reaction IV, can be further derivatized to hydrophobic, cationic, and anionic *ortho*-naphthoquinones by reaction with mono-acids, amino acids, or di-acids, as shown in Reactions VI, VII, and VIII, respectively.

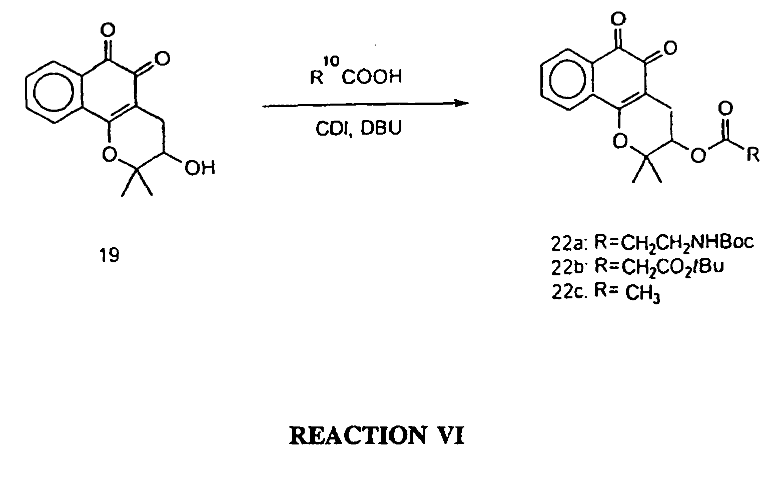

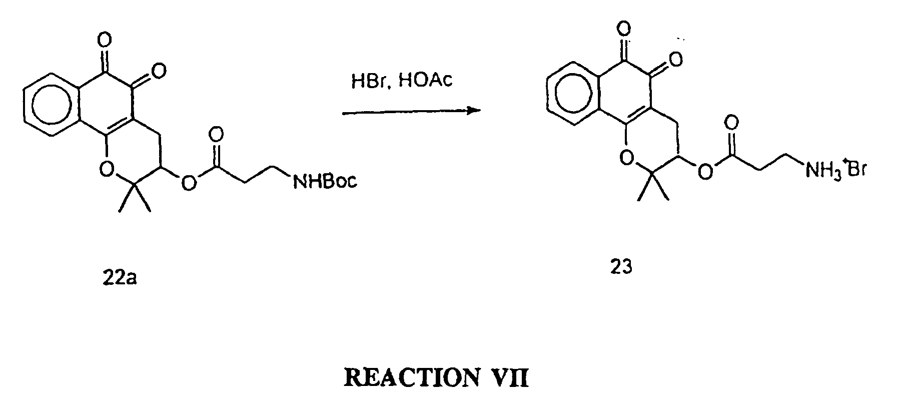

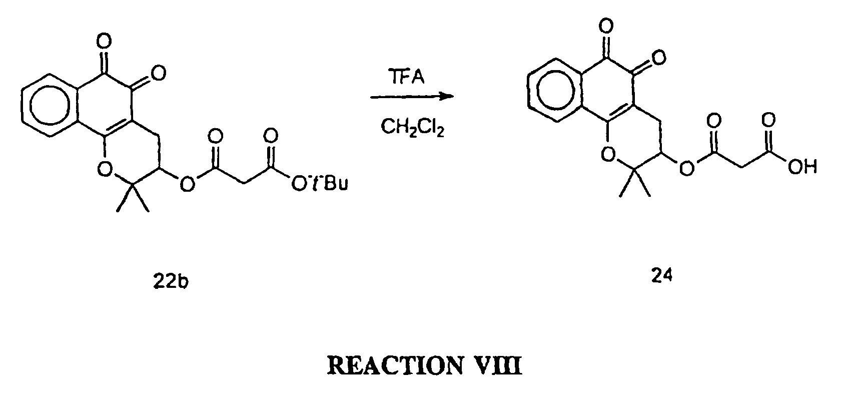

Reaction VI depicts the synthesis of mono-acid derivatives, such as the 3-*O-*acetyl derivative (22c). Reaction VII illustrates the synthesis of amino acid ester derivatives, such as the 3-*O*-alanyl derivative (23). Reaction VIII shows the synthesis of di-acid derivatives, such as the 3-*O*-malonyl derivative (24).

The preferred method to accomplish the transformation shown in Reaction VI is to react the 3-hydroxy-β-lapachone with an acid, R¹⁰COOH, and the corresponding carbonyldimidazole, CDI, in the presence of a non-nucleophilic base. As illustrated here, DBU (*i.e.,* 1,8-diazabicyclo(5.4.0)undec-7-ene) is used. DBU is preferred. However, several equivalent reagents are known to those skilled in the art. Reaction VI can be used to generate, *inter alia,* carbonyl and di-carbonyl derivatives.

Reaction VII illustrates the conversion of a monoacid formed in Reaction VI into an amino acid derivative. As shown, the conversion to an amino acid salt is accomplished by treatment with HBr in a suitable solvent (*e.g.*, diethyl ether).

Reaction VIII illustrates the synthesis of a di-acid derivative by treating a *t-*butyl ester generated in Reaction VI with a strong acid.

Examples and a further description of Reaction VI, VII, and VIII are included in the Examples section, below.

### Synthesis of 4-Substituted-1,2-Napthoquinones

Synthesis of these compounds can be accomplished using standard literature methodologies. The starting materials, 1,2-naphthoquinone and lawsone, and the necessary reagents, are widely available from several international commercial suppliers, for instance the Sigma Chemical Company (St. Louis, Missouri, USA).

Preparation of 4-alkoxy-1,2-naphthoquinones: The synthesis of 4-alkoxy-1,2-naphthoquinones proceeds via the silver salt of lawsone. The preferred synthesis is that described by *L.F. Fieser* (1926 and 1927). An equally suitable synthesis is described by *Takuwa et al.* (1986). An illustrative synthesis, showing the preparation of 4-isopropoxy-1,2-naphthoquinone, proceeds as follows:

To 750 mL of hot water was added 50.0 g (0.287 mol) of 2-hydroxy-1,4-naphthoquinone. Sufficient ammonium hydroxide was added to bring the quinone into solution. Then 287 mL of 1 M AgNO₃ was added, and the mixture was cooled in an ice-bath. The red salt was collected by filtration and washed successively with water, ethanol, and diethyl ether and dried. Yield: 76.13 g (94%) of silver salt.

To a suspension of 5.00 g (17.8 mmol) of the silver salt of 2-hydroxy-1,4-naphthoquinone in 50 mL of benzene was added 1.85 mL (19.7 mmol) of 2-bromopropane. The reaction mixture was heated at 50°C for 24 hr. The benzene was removed under reduced pressure. The crude material was treated with ethyl acetate to dissolve the organic products, and the resultant solution was filtered to remove silver salts. The ethyl acetate solution was extracted first with cold 10% NH₄OH until the extract was colorless, and then with 5% NaHSO₃ until the extract gave no precipitate of orthoquinone upon addition of Na₂CO₃ solution. The NaHSO₃ extracts were combined, treated with Na₂CO₃ solution, and extracted with CH₂Cl₂. The CH₂Cl₂ extracts were combined and dried with MgSO₄. Removal of solvent yielded 2.03 g (53%) of 4-(sopropoxy-1,2-naphthoquinone, which was recrystallized from benzene-ligroin: mp 125-125.5 °C; ¹H NMR (300 MHz, CDCl₃, TMS) δ 8,12 (dd, J = 8.1 Hz, 1H), 7.89 (dd, J = 8.1 Hz, 1H), 7.69 (td, J = 8.1 Hz, 1H), 7.59 (td, J = 8.1 Hz, 1H), 5.96 (s, 1H), 4.75 (septet, J = 6 Hz, 1H), 1.49 (d, J = 6 Hz, 6H); ¹³C NMR (75.5 MHz, CDCl₃, TMS) δ 179.5, 179.3, 166.7, 134.8, 132.3, 131.3, 130.4, 128.6, 124.8, 103.7, 72.9, 21.4. Analysis calculated for C₁₃H₁₂O₃: C = 72.20, H = 5.60; Found: C = 72.06, H = 5,62.

Preparation of 4-thio-1,2-naphthoquinones: The synthesis of the thiols of Formula I can be effected using the method of *Mackenzie et al.* (1986). An illustrative synthesis for the preparation of 4-pentanethio-1,2-naphthoquinone, proceeds as follows:

To a solution of 1.00 g (6.32 mmol) of 1,2-naphthoquinone in 150 mL of methanol was added 784 µL (6.32 mmol) of pentanethiol. The reaction mixture was stirred at room temperature for 22 hr. Removal of the methanol and column chromatography on silica gel with CH₂Cl₂ afforded 104.2 mg (6%) of 4-pentanethio-1,2-naphthoquinone, which was recrystallized from hexanes: mp 107-108°C; ¹H NMR (300 MHz, CDCl₃, TMS) δ 8.17 (d, J = 7.7 Hz, 1H), 7.85 (d, J = 7.7 Hz, 1H), 7.68 (t, J = 7.7 Hz, 1H), 7.57 (t, J = 7.7 Hz, 1H), 6.42 (s, 1H), 3.04 (t, J = 7 Hz, 2H), 1.9-1.8 (m, 2H), 1.5-1.3 (m, 4H), 0.95 (t, J = 7 Hz, 3H); ¹³C NMR (75.5 MHz, CDCl₃, TMS) δ 179.6, 176.2, 160.2, 134.9, 133.7, 131.1, 130.4, 129.2, 125.2, 119.7, 31.8, 31.1, 27.2, 22.2, 13.8. Analysis calculated for C₁₅H₁₆O₂S: C= 69.19, H = 6.21, S = 12.31; Found C = 68.97, H = 6.22, S = 12.31.

Preparation of 4-amino-1,2-naphthoquinones: The amino derivatives can be obtained via reaction of the alkoxy derivatives with an appropriate amine. The preferred synthetic route is that described by *L.F. Fieser* (1935). An illustrative synthesis of 4-pentylamino-1,2-naphthoquinone proceeds as follows:

To a stirred suspension of 650 mg (3.45 mmol) of 4-methoxy-1,2-naphthoquinone in 20 mL of ethanol was added 0.80 mL (6.90 mmol) of pentylamine. The reaction mixture was stirred at room temperature for 60 min. The resulting red precipitate was collected by filtration and then triturated with 20 mL of ethyl acetate to afford 411.7 mg (49%) of 4-pentylamino-1,2-naphthoquinone, which was recrystallized from ethyl acetate: mp > 215°C; ¹H NMR (300 MHz, CDCl₃, TMS) δ 8.21 (dd, J = 7.5, 1.6 Hz, 1H), 7.69 (td, J = 7.7, 1.6 Hz, 1H), 7.61 (t, J = 7.5 Hz, 1H), 7.49 (d, J = 7.9 Hz, 1H), 5.86 (s, 1H), 5.76 (br s, 1H), 3.4-3.3 (m, 2H), 1.8-1.7 (m, 4H), 1.5-1.4 (m, 4H), 0,95 (t, J = 7 Hz, 3H); ¹³C NMR (75.5 MHz, d₆₋DMSO, TMS) δ 182.0, 174.7, 154.8, 134.1, 131.2, 130.8, 127.8, 123.3, 98.0, 43.1, 28.7, 27.4, 21.8, 13.9. Analysis calculated for C₁₅H₁₇NO₂: C = 74.04, H = 7.06, N = 5.7; Found: C = 73.88, H = 7.01, N = 5.73.

### Definitions

As used hereinbelow, the terms "cancer," "cancer cell(s)," and "neoplasm(s)" are synonymous and refer to all types of cancer, including both benign and malignant tumors and neoplasms, without limitation. This includes melanomas, lymphomas, leukemias, sarcomas, and the like. Illustrative examples of cancerous tumors and tissues are cutaneous tumors, such as malignant melanomas and mycosis fungoides; hematologic tumors, such as leukemias, for example, acute lymphoblastic, acute myelocytic or chronic myelocytic leukemia; lymphomas, such as Hodgkin's disease or malignant lymphoma; gynecologic tumors, such as ovarian and uterine tumors; urologic tumors, such as those of the prostate, bladder, or testis; soft tissue sarcomas, osseus or non-osseus sarcomas, breast tumors; tumors of the pituitary, thyroid, and adrenal cortex; gastrointestinal tumors, such as those of the esophagus, stomach, intestine, and colon; pancreatic and hepatic tumors; laryngeae papillomestasas, brain and other CNS tumors, and lung tumors.

"ADR-resistant" designates cancer cells which are resistant to the antineoplastic drug referred to as adriamycin and other antineoplastic drugs which share the same skeletal structure as adriamycin, such as daunorubicin and teniposide. Although now generally referred to in the literature as doxorubicin (and also known as adriablastina), because the term "adriamycin" is still commonly used, it shall be used herein. "ADR" shall be used to designate adriamycin itself. Adriamycin, daunorubicin, and teniposide are cytotoxic agents believed to exert their cytotoxicity via their ability to intercalate into DNA. ADR-resistant cells are those cells which are specifically resistant to the cytotoxicity of adriamycin, and which may have some degree of resistance to daunorubicin or other chemotherapeutic compounds. The systematic name of adriamycin is (8S-*cis*)-10-[(3-amino-2,3,6-trideoxy-α-L-lyxo-hexapyrahosyl)oxy] -7,8,9,10-tetrahydro-6,8,11-trihydroxy-8-(hydroxyacetyl)-1-methoxy-5,12-naphthacenedione.

"MDR" refers to multi-drug resistant cancer cells. As defined herein, these cancers are neoplastic cells are resistant to all chemotherapeutic agents now commonly used in the chemical treatment of cancers.

"Merbarone" is the common name given to 5-(N-phenylcarboxamido)-2-thiobarbituric acid.

"Inhibiting cell growth" means slowing, interrupting, arresting, and/or terminating the growth, proliferation, and/or metastases of a rapidly proliferating cancer cell or cancerous cell line. It is understood that inhibiting cell growth of a cancer cell or cell line does not necessarily provide a "cure" in the sense that the tumor tissue or transformed cells are completely destroyed.

### Biological Activity of the Tricyclic o-Naphthoquinones

Of great significance in the present invention is the utility of the described naphthoquinones to inhibit the growth and proliferation of neoplastic cells. Further still, in standard *in vitro* testing, the naphthoquinones described herein induce cell death in several neoplastic cell lines at drug concentrations smaller than 10 µM. The tricyclic naphthoquinones of the present invention have been shown to cause cell death in accepted *in vitro* test cultures for human breast cancer, lung cancer, colon cancer, and prostate cancer at minute concentrations heretofore undescribed in the scientific literature.

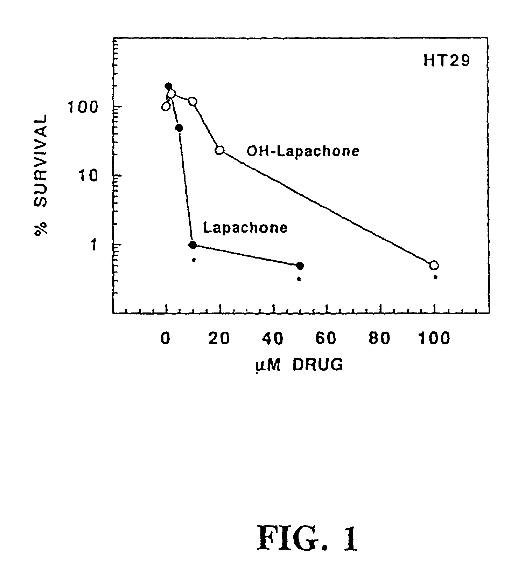

Figs. 1-8 illustrate a series of experiments designed to illustrate the ability of the tricyclic o-naphthoquinones to induce cell death in standard neoplastic cell lines. Each graph of Figs. 1-8 has as its X-axis the concentration of the particular compound being tested. The Y-axis of each graph is a semi-logarithmic scale of at least 4 orders of magnitude representing the fraction of cell survival in each of the cultures tested. A standardized protocol was used throughout all of the test cultures. The test protocol and neoplastic cell lines tested, as well as a complete description of each of the graphs shown in Figs. 1-8 follows:

### Cell Culture and Drug Testing Protocol

For each of the *in vitro* tests whose results are depicted in the Figures, the following standard protocol was followed.

### Day 1:

10 standard culture flasks for each drug to be tested are plated with 5 x 10⁵ cells in 5 mL of media and allowed to incubate for 16-24 hours at 37°C.

### Day 2:

Fresh stocks of the compounds to be evaluated are prepared in sterile DMSO. For each drug, two of the ten culture flasks prepared on Day 1 are used as controls. The control flasks are treated with DMSO only. Four flasks for each compound are then treated with serially-diluted concentrations of the compound (1, 5, 10,50 µm or 2, 10, 20, 100 µm). The remaining flasks are left untouched. The cells are incubated for 4 hours at 37°C.

After 4 hours the control flasks are counted (2 counts for each flask) and the cells per mL calculated based on the average of the control counts. The cells are then re-plated into six 60 mm dishes for each flask from dilutions based on the cells/mL of the control. (In the various test runs, cell concentrations ranged from approximately 50 to approximately 800 cells per mL.)

### Day 15-20:

The cells are monitored for colony formation. When visible, the cells are stained with 0.5% crystal violet (in 95% EtOH) and counted. The plating efficiency for each dish is then calculated. The plating efficiencies of the six dishes for each flask are averaged and the standard deviation is calculated. The fraction of cell survival at each concentration is determined based on the controls and plotted as log fraction of cell survival ± standard deviation versus the dose of the compound.

The ED₅₀ results of the testing, and the respective figures which graphically illustrate the test results are summarized as follows:

**Colony Experiments**

| Cell Line | ED₅₀ Values | |
|---|---|---|
| HT29 | ED₅₀ (Lap) = 4.8 µM | Fig. 1 |
| | ED₅₀ (OH-Lap) = 15.4 µM | |
| | | |
| BE | ED₅₀ (Lap) = 8 µM | Fig. 2 |
| | ED₅₀ (OH-Lap) = 0.6 µM | |
| | | |
| A549 | ED₅₀ (Lap) = 6.1 µM | Fig. 3 |
| | ED₅₀ (OH-Lap) = 18 µM | |
| | | |
| A549 | ED₅₀ (Lap) = 5.8 µM | Fig. 4 |
| | ED₅₀ (Drug A) = 6 µM | |
| | | |
| A549 | ED₅₀ (Drug B) = 5.6 µM | Fig. 5 |
| | ED₅₀ (Drug C) = 4.3 µM | |
| | | |
| MCF7 | ED₅₀ (Lap) = 9.8 µM | Fig. 6 |
| | ED₅₀ (OH-Lap) = 7.7 µM | |
| | | |
| MCF7 | ED₅₀ (Lap) = 1.6 µM | Fig. 7 |
| | ED₅₀ (Drug A) = 1.6 µM | |
| | | |
| MCF7 | ED₅₀ (Drug B) = 1.4 µM | Fig. 8 |
| | ED₅₀ (Drug C) = 1.4 µM | |

### Effectiveness against colon cancer:

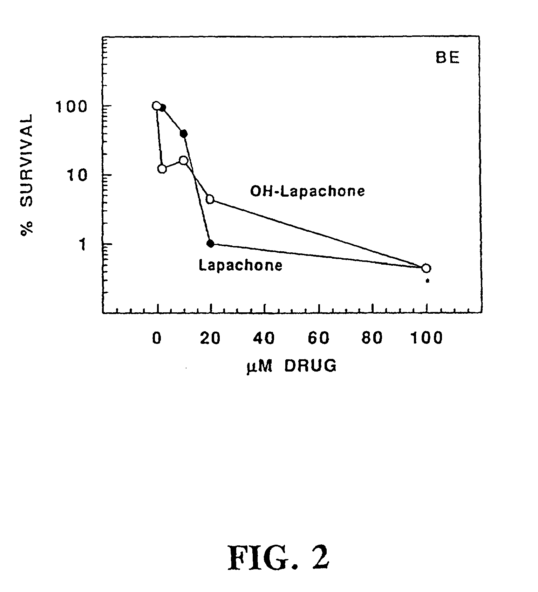

The cell lines HT29 and BE are both human colon cancer cell lines which are used to test the effectiveness of a given agent against cancer cell growth and proliferation. Figures 1 and 2 depict the cell survival in cultures of HT29 and BE upon the addition of β-lapachone (●) and 3-hydroxy-β-lapachone (○). The HT29 and BE cell cultures were prepared and tested according to the protocol given above.

As clearly shown in Figures 1 and 2, at a concentration of approximately 10 µM, β-lapachone already significantly impacts upon the ability of the HT29 and BE cells to reproduce. 3-Hydroxy-β-lapachone also significantly limits the reproductive ability of these cancer cell lines.

In Fig. 1, the ED₅₀ value for β-lapachone is 4.8 µM, and the ED₅₀ for 3-hydroxy-β-lapachone is 15.4 µM. (Bear in mind that the Y-axes in Figs. 1-8 are logarithmic, not linear.)

For the BE cell line tested, the ED₅₀ for the β-lapachone plot shown in Fig. 2 is 8 µM, while the ED₅₀ for 3-hydroxy-β-lapachone is 0.6 µM.

The numerical values and standard deviations of the individual data points presented in Figs. 1 and 2 are tabulated in Tables 1 and 2, respectively.

**Table 1: Fraction of Cell Survival HT29 With β-Lapachone and 3-Hydroxy-β-lapachone (Fig. 1)**

| Concentration of Drug (µM) | Fraction of Cell Survival | Standard Deviation |
|---|---|---|
| 0 | 0.9868 | 0.0935 |
| 1 | 0.9669 | 0.1228 |
| 5 | 0.4901 | 0.0480 |
| 10 | 0.0000 | 0.0000 |
| 50 | 0.0000 | 0.0000 |
| | | |
| 0 | 1.0132 | 0.0688 |
| 2 | 1.5232 | 0.1125 |
| 10 | 1.1854 | 0.0532 |
| 20 | 0.0232 | 0.0020 |
| 100 | 0.0000 | 0.0000 |

**Table 2: Fraction of Cell Survival BE With β-lapachone and 3-Hydroxy-β-lapachone (Fig. 2)**

| Concentration of Drug (µM) | Fraction of Cell Survival | Standard Deviation |
|---|---|---|
| 0 | 1.1111 | 0.0731 |
| 2 | 0.0117 | 0.0082 |
| 10 | 0.1579 | 0.0126 |
| 20 | 0.0439 | 0.0102 |
| 100 | 0.0000 | 0.0000 |
| | | |
| 0 | 0.8889 | 0.0850 |
| 2 | 0.9293 | 0.1280 |
| 10 | 0.3918 | 0.0627 |
| 20 | 0.0102 | 0.0046 |
| 100 | 0.0000 | 0.0000 |

### Effectiveness against lung cancer:

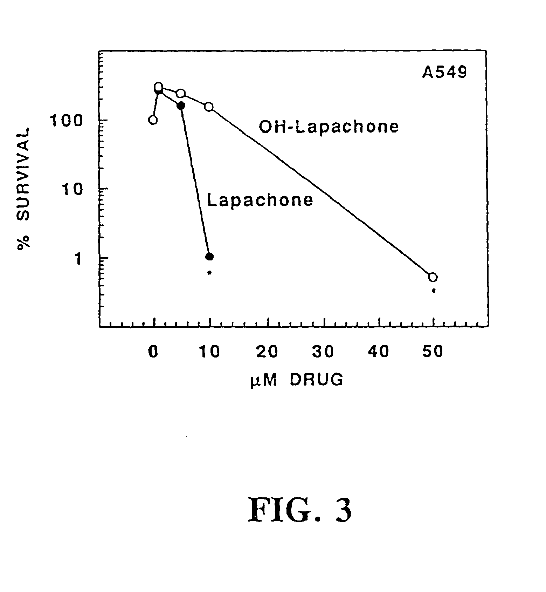

Figs. 3, 4, and 5 depict the results of studies of the effectiveness of several different tricyclic *o*-naphthoquinones against lung cancer cells A549. The compounds tested were β-lapachone, 3-hydroxy-β-lapachone, 3,3-DINOR-dunnione (designated "Drug A" in the figures), 3-NOR-dunnione (designated "Drug B" in the figures) and dunnione itself (designated "Drug C" in the figures). A legend for the figures is provided below. The cell lines were cultured and the compounds evaluated according to the standard protocol described above.

**FIGURE LEGEND**

| **Designation** | **Formula** | **Trivial Name** |
|---|---|---|
| Drug A | 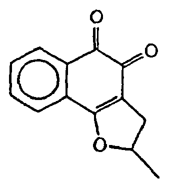 | 3,3-DINOR-dunnione |
| Drug B | 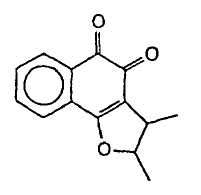 | 3-NOR-dunnione |
| Drug C | 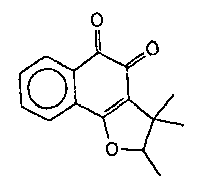 | dunnione |
| Drug D | 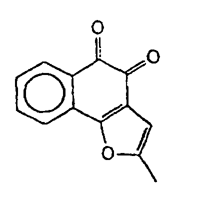 | 3,3-DINOR-2,3-dehydrodunnione |

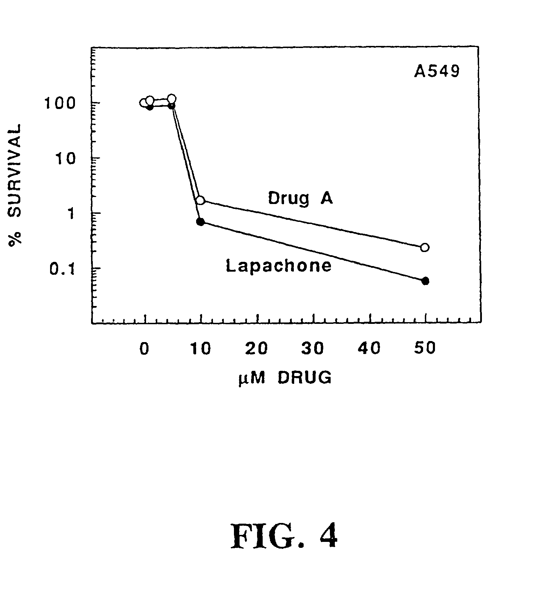

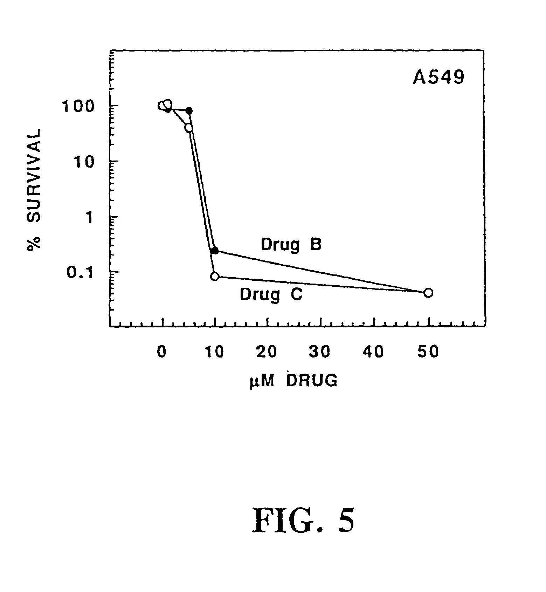

All of the compounds tested exhibited excellent ED₅₀ values against the proliferation of A549 lung cancer cells. For instance, in Fig. 3, the ED₅₀ for β-lapachone (●) is 6.1 µM, and the ED₅₀ for 3-hydroxy-β-lapachone (O) is 18 µM. The data in Fig. 4 indicate that ED₅₀ (β-lapachone) (●) = 5.8 µM, and ED₅₀ (3,3-DINOR-dunnione) (○) = 6 µM. ED₅₀ (3-NOR-dunnione) (●) = 5.6 µM in Fig. 5, and the ED₅₀ for dunnione (○) itself is a remarkably low 4.3 µM.

The numerical values and standard deviations of the individual data points presented in Figs. 3, 4, and 5 are tabulated in Tables 3-8, below:

**Table 3: Fraction of Cell Survival A549 With β-Lapachone (Fig. 3)**

| Concentration of Drug (µM) | Fraction of Cell Survival | Standard Deviation |
|---|---|---|
| 0 | 1.5556 | 0.0327 |
| 1 | 2.6528 | 0.0667 |
| 5 | 1.5833 | 0.0899 |
| 10 | 0.0000 | 0.0000 |
| 50 | 0.1771 | 0.1017 |

**Table 4: Fraction of Cell Survival A549 With 3-Hydroxy-β-lapachone (Fig. 3)**

| Concentration of Drug (µM) | Fraction of Cell Survival | Standard Deviation |
|---|---|---|
| 0 | 0.4444 | 0.0197 |
| 1 | 3.0417 | 0.0978 |
| 5 | 2.3924 | 0.0637 |
| 10 | 1.5330 | 0.0431 |
| 50 | 0.0000 | 0.0000 |

**Table 5: Fraction of Cell Survival A549 With β-Lapachone (Fig. 4)**

| Concentration of Drug (µM) | Fraction of Cell Survival | Standard Deviation |
|---|---|---|
| 0 | 1.0161 | 0.0818 |
| 1 | 0.8552 | 0.0580 |
| 5 | 0.8966 | 0.0303 |
| 10 | 0.0069 | 0.0027 |
| 50 | 0.0000 | 0.0000 |

**Table 6: Fraction of Cell Survival A549 With "Drug A" (3,3-DINOR-Dunnione) (Fig. 4)**

| Concentration of Drug (µM) | Fraction of Cell Survival | Standard Deviation |
|---|---|---|
| 0 | 0.9840 | 0.0585 |
| 1 | 1.1080 | 0.0705 |
| 5 | 1.2115 | 0.0238 |
| 10 | 0.0172 | 0.0044 |
| 50 | 0.0023 | 0.0006 |

**Table 7: Fraction of Cell Survival A549 With "Drug B" (3-NOR-Dunnione) (Fig. 5)**

| Concentration of Drug (µM) | Fraction of Cell Survival | Standard Deviation |
|---|---|---|
| 0 | 1.0962 | 0.0724 |
| 1 | 0.8972 | 0.1169 |
| 5 | 0.8190 | 0.0950 |
| 10 | 0.0024 | 0.0031 |
| 50 | 0.0000 | 0.0000 |

**Table 8: Fraction of Cell Survival A549 With "Drug C" (Dunnione) (Fig. 5)**

| Concentration of Drug (µM) | Fraction of Cell Survival | Standard Deviation |
|---|---|---|
| 0 | 0.9088 | 0.1003 |
| 1 | 1.0962 | 0.0888 |
| 5 | 0.3997 | 0.0284 |
| 10 | 0.0000 | 0.0000 |
| 50 | 0.0000 | 0.0000 |

### Effectiveness against breast cancer.

In the same fashion as the above tests, several lapachone and dunnione derivatives were evaluated for their efficacy in inhibiting the proliferation of breast cancer cells. In this instance, the cancer cell line utilized was MCF7.

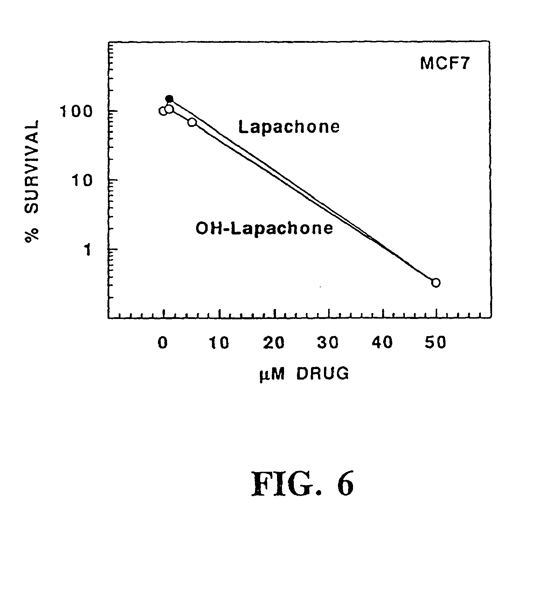

Fig. 6 depicts the fraction of cell survival for a series of MCF7 cell cultures exposed to β-lapachone (●) and 3-hydroxy-β-lapachone (○). Here, the ED₅₀ for β-lapachone was found to be 9.8 µM, while the ED₅₀ for 3-hydroxy-β-lapachone was found to be 7.7 µM.

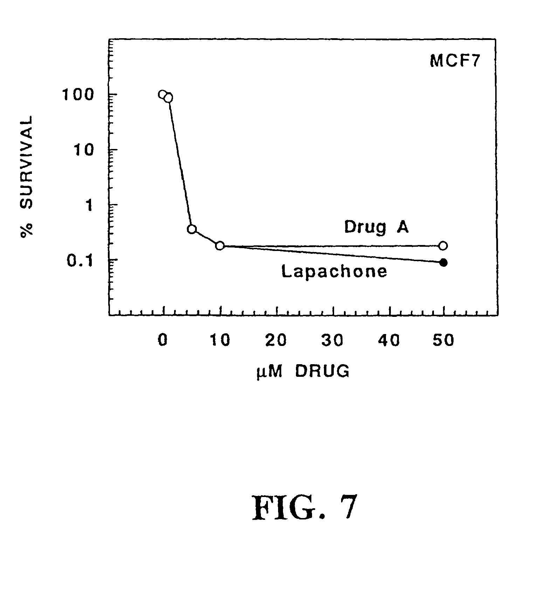

Fig. 7 depicts a comparative cell survival study between β-lapachone (●) and 3,3-DINOR-dunnione ("Drug A") (○). In this study, the ED₅₀ for both β-lapachone and 3,3-DINOR-dunnione was found to be a very low 1.6 µM.

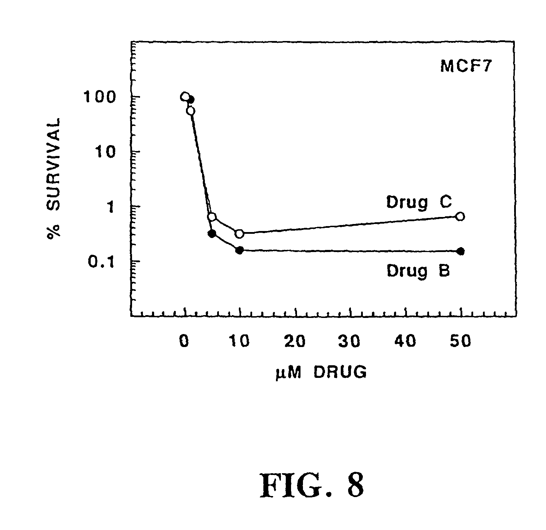

Fig. 8 depicts an identical comparative cell survival study between 3-NOR-dunnione ("Drug B") (●) and dunnione itself ("Drug C") (○). The ED₅₀ levels in this study were also shown to be remarkably low. For both the 3-NOR-dunnione and dunnione itself, the ED₅₀ was found to be 1.4 µM.

The numerical values and standard deviations of the individual data points presented in Figs. 6, 7, and 8 are tabulated in Tables 9-14, below.

**Table 9: Fraction of Cell Survival MCF7 With β-Lapachone (Fig. 6)**

| Concentration of Drug (µM) | Fraction of Cell Survival | Standard Deviation |
|---|---|---|
| 0 | 0.5770 | 0.0152 |
| 1 | 1.5 | 0.2665 |
| 5 | 0.0000 | 0.0000 |
| 10 | 0.0000 | 0.0000 |
| 50 | 0.0032 | 0.0010 |

**Table 10: Fraction of Cell Survival MCF7 With 3-Hydroxy-β-lapachone (Fig. 6)**

| Concentration of Drug (µM) | Fraction of Cell Survival | Standard Deviation |
|---|---|---|
| 0 | 1.4231 | 0.0423 |
| 1 | 1.0641 | 0.0553 |
| 5 | 0.6795 | 0.0218 |
| 10 | 0.0000 | 0.0000 |
| 50 | 0.0032 | 0.0010 |

**Table 11: Fraction of Cell Survival MCF7 With β-Lapachone (Fig. 7)**

| Concentration of Drug (µM) | Fraction of Cell Survival | Standard Deviation |
|---|---|---|
| 0 | 1.0145 | 0.0783 |
| 1 | 0.9197 | 0.0687 |
| 5 | 0.0036 | 0.0020 |
| 10 | 0.0018 | 0.0010 |
| 50 | 0.0009 | 0.0005 |

**Table 12: Fraction of Cell Survival MCF7 With "Drug A" (3,3-DINOR-Dunnione) (Fig. 7)**

| Concentration of Drug (µM) | Fraction of Cell Survival | Standard Deviation |
|---|---|---|
| 0 | 0.9854 | 0.0579 |
| 1 | 0.8540 | 0.0389 |
| 5 | 0.0036 | 0.0020 |
| 10 | 0.0018 | 0.0010 |
| 50 | 0.0018 | 0.0005 |

**Table 13: Fraction of Cell Survival MCF7 With "Drug B" (3-NOR-Dunnione) (Fig. 8)**

| Concentration of Drug (µM) | Fraction of Cell Survival | Standard Deviation |
|---|---|---|
| 0 | 0.8377 | 0.0593 |
| 1 | 0.0888 | 0.0444 |
| 5 | 0.0032 | 0.0020 |
| 10 | 0.0016 | 0.0010 |
| 50 | 0.0154 | 0.0035 |

**Table 14: Fraction of Cell Survival MCF7 With "Drug C" (Dunnione) (Fig. 8)**

| Concentration of Drug (µM) | Fraction of Cell Survival | Standard Deviation |
|---|---|---|
| 0 | 1.1624 | 0.0781 |
| 1 | 0.5584 | 0.0674 |
| 5 | 0.0065 | 0.0041 |
| 10 | 0.0032 | 0.0013 |
| 50 | 0.0065 | 0.0013 |

### Effectiveness against prostate cancer:

Additional biological testing was conducted to evaluate the efficacy of the tricyclic *o*-naphthoquinones against prostate cancer and to further evaluate the biological activity of the compounds against breast cancer. The compounds evaluated were as follows:
Drug B = 3-NOR-Dunnione
Drug C = Dunnione
Drug D = 3,3-DINOR-2,3-Dehydrodunnione

Table 15 lists the IC₅₀ determinations of Drugs B, C, and D against the human prostate cancer cell line PC-3 and the human breast cancer cell line MCF7.

**Table 15: IC₅₀ Determinations of Drugs B, C, and D on Human Prostate (PC-3) and Breast (MCF7) Cell Lines**

| Structures | IC₅₀ (µM) | |
|---|---|---|
| Naphtho(2,3-b)dihydrofurandiones | PC-3 | MCF7 |
| Drug B | 0.7 | 0.5 |
| Drug C | 0.6 | 0.7 |
| Drug D | 0.9 | 0.7 |

IC₅₀ calculations for each cell line were determined by DNA amount and anchorage-dependent colony formation (CF) as described elsewhere. (See Planchon et al. *Cancer Res.* **55**, 3706 (1995), incorporated herein by reference.) In short, IC₅₀ calculations for each cell line were determined by DNA amount and anchorage-dependent colony formation (CF) assays. For the CF assay, cells were seeded at 500 viable cells/well in 6-well plates and incubated overnight, then treated with equal volumes of media containing β-lapachone at final concentrations ranging from 0.005 to 50 µM in half-log increments (controls were treated with 0.25 % DMSO, equivalent to the highest dose of β-lapachone used) for 4 hours or for continuous 12 hour exposures. Plates (3 wells/condition) were stained with crystal violet, and colonies of > 50 normal-appearing cells were enumerated. IC₅₀ values for various cells were calculated using drug doses with numbers of colonies surrounding 50% of control. For DNA assays, plates were harvested for IC₅₀ determinations 8 days after treatment using a CytoFluor 2350 fluorescence measurement system (Millipore). Six-well samplings were included in the calculation of DNA fluor units for each dose. A graph of β-lapachone dose *versus* percentage control DNA in fluor units was used to calculate each IC₅₀. The cells were exposed for 24 hours to the tricyclic naphthoquinones. All experiments were repeated at least twice, each in duplicate. PC-3 is an androgen-independent prostate cancer cell line.

To further study the presently described compounds, their ability to induce apoptosis (programmed cell death) in human prostate and breast cancer cell lines was evaluated. The results are encouraging in the all of the compounds tested induced apoptosis at a concentration of 5 µM.

**Table 16: Apoptopic Effects of B, C, and D on Human Prostate (PC-3) and Breast (MCF7) Cancer Cell Lines**

| **Structures** | **Apoptosis Observed** | | | | | |
|---|---|---|---|---|---|---|
| | | | | | | |

| **Naphtho (2,3-b) dihydrofurandiones** | **Concentrations** | | | | | |
|---|---|---|---|---|---|---|
| | 1 µm | 5 µm | 10 µm | 25 µm | 50 µm | 100 µm |
| Drug C | - | + | + | + | + | + |
| Drug B | + | + | + | + | + | + |
| Drug D | + | + | + | + | + | + |

Quantification of apoptotic cells and alterations in cell cycle distribution were determined 24 hours after drug treatment (1.0 - 100 µm; 4 h) by flow cytometry and DNA laddering as described by Planchon et al. *supra.* Experiments were repeated at least three times, each in duplicate. The above results apply to both the PC-3 and MCF7 cell lines.

An illustrative method to determine apoptosis proceeds as follows: Cells (1 X 10⁶/condition) were treated with or without various concentrations of β-lapachone, topotecan, or camptothecin for various times. Trypsinized or pelleted cells were washed with ice-cold Tris/saline solution (10 mM Tris (pH 7.0) and 50 mM NaCI), fixed in 90% ethanol-Tris/saline, and stored at -4°C. Cells were washed with phosphate-citric acid buffer (0.2M Na₂HPO₄ and 0.1 M citric acid (pH 7.8)) and stained with a solution containing 0.2 % NP40, RNase A (7000 units/ml), and 33 µg/ml propidium iodide at 4°C for 10 minutes. Stained nuclei were then analyzed for DNA-propidium fluorescence using a Becton Dickinson FACScan (San Jose, CA) at a laser setting of 36 mW and an excitation wavelength of 488 nm. Resulting DNA distributions were then analyzed for proportion cells in apoptosis, G₀/G₁, S, and G₂/M of the cell cycle. Data was analyzed by ModFit (Verity Software House, Inc., Topsham, ME). All experiments were repeated at least three times, each in duplicate.

Cells from the above conditions were also analyzed for the formation of 180-200-bp DNA laddering, which can be diagnostic for certain cells undergoing apoptosis. Treated and control cells were washed twice with PBS containing 1 mM EDTA at ambient temperature and lysed in 10 mM EDTA, to mM Tris-HCl (pH 8.0), 0.5% (w.v) sodium lauryl sarkosinate, and 0.5 mg/ml RNase A for at least 1 hour at 37°C and then with 1.0 mg/ml proteinase K at 37°C for at least 1 hour. Loading buffer (10 mM EDTA, 1% (w/v) low melting point agarose, 0.25% (w.v) bromophenol blue, and 40% (w.v) sucrose) was then added (10% final concentration), and heated (70°C) samples were loaded onto presolidified, 1.8% (w/v) agarose gels containing 0.1 µg/ml ethidium bromide using end-cut Rainin (Woburn, MA) 1-ml pipette tips to avoid DNA shearing. Agarose gels were run at 65 V/cm for 10 minutes and then at 15 V/cm overnight in 1 x TAE (1.0 M Tris-acetate (pH 7.5) and 10 mM EDTA) running buffer.

### Inhibition of Topoisomerase I and II:

Once it had been discovered that the tricyclic compounds described herein would not only inhibit cell growth, but would actively induce cell death via apoptosis, a mechanism to account for these biological effects was investigated. As shown in Table 17, it was found that the compounds of the present invention are inhibitors of Topoisomerase I (Topo I). By inhibiting the function of Topo I, which catalyzes the unwinding of DNA strands prior to replication, it is hypothesized that the compounds described above induce cell death by preventing access to the genetic information necessary to carry on normal cellular operations.

**Table 17: Inhibition of the Catalytic Activity of Topoisomerase I by Drugs B, C, and D**

| **Structures** | **Inhibition of Topoisomerase I** | | | |
|---|---|---|---|---|
| | | | | |

| Naphto (2,3-b) furan diones | 1 µm | 10 µm | 100 µm | 1 mM |
|---|---|---|---|---|
| Drug C | - | + | + | + |
| Drug B | + | + | + | + |
| Drug D | - | - | + | + |

Topoisomerase I (Topo I) from human placenta (3.0 units) was incubated with various concentrations of Drug B, C, or D for 10 min. at 37°C. p36B4 Supercoiled plasmid DNA (1.5 µg) was then added to initiate DNA unwinding reactions. Topo I DNA unwinding activity was measured as described in Hsiang et al. *J. Biol. Chem.,* **260**, 14873 (1985), incorporated herein by reference.

Topoisomerase I enzymatic activity can be assayed in the following manner: supercoiled DNA unwinding assays using purified human placenta Topo I (TopoGEN, Inc., Columbus, OH) were performed with or without drug addition to assess the inhibitory effects of β-lapachone, camptothecin, and topotecan under various reaction conditions. Enzymatic assays were performed in two basic fashions. In the first reaction sequence, Topo I (3.0 units) was incubated with increasing concentrations of β-lapachone, camptothecin (or topotecan), or DMSO for 5 minutes at 37°C in Topo I reaction buffer (without dATP). p36B4 Supercoiled DNA (1.5 µg) was then added to begin the reactions, and aliquots were taken at various times. In the second reaction sequence, p364B4 DNA (1.5 µg) was incubated with various β-lapachone, camptothecin, or topotecan concentrations for 5 minutes at 36°C, and Topo I (3.0 units) was added at *t* = 0. Aliquots were removed at various times to follow DNA unwinding reactions and immediately treated with SDS-proteinase K at 65°C and loaded onto 0.7% agarose gels; supercoiled (form 1) substrate was separated and quantified from reaction intermediates (R) and open circular (form II) product. On most agarose gels, DNA molecular weight markers (λ DNA cut from *Eco*RI*-Hind*III (marked "Lambda *Eco*RI DNA Marker"); Sigma Chemical Co., St. Louis, MO), linearized p36B4 plasmid DNA (cut with *Pst*I), and p36B4 plasmid DNA substrate were concomitantly added. Gels were stained with 50 µg/ml ethidium bromide and destained for 30 minutes in distilled water; the loss of form I relative to total DNA loaded was quantified by densitometric scans of photographic negatives (type 55; Polaroid, Cambridge, MA). Enzyme inhibition was defined as the effects of various drugs on Topo I activity compared to control (DMSO alone) reactions.

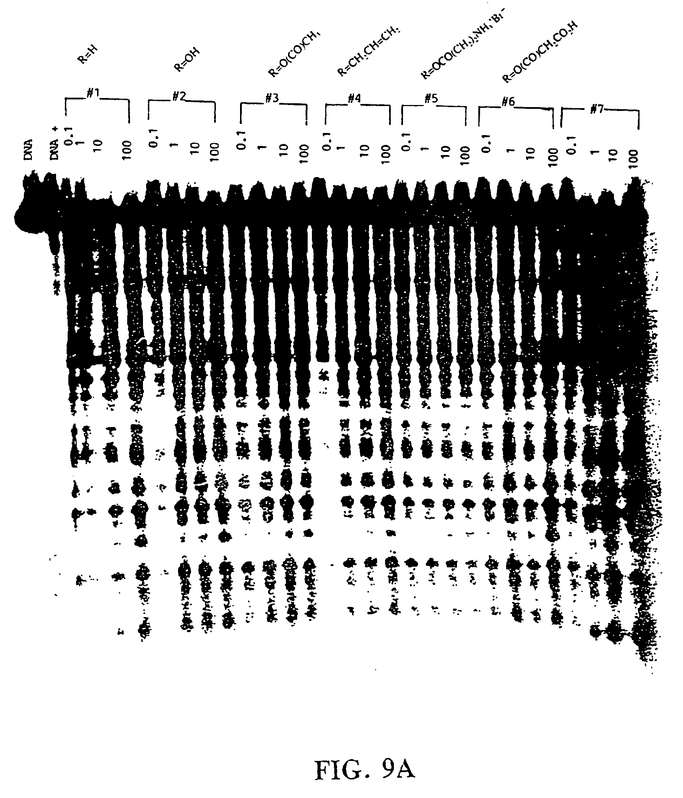

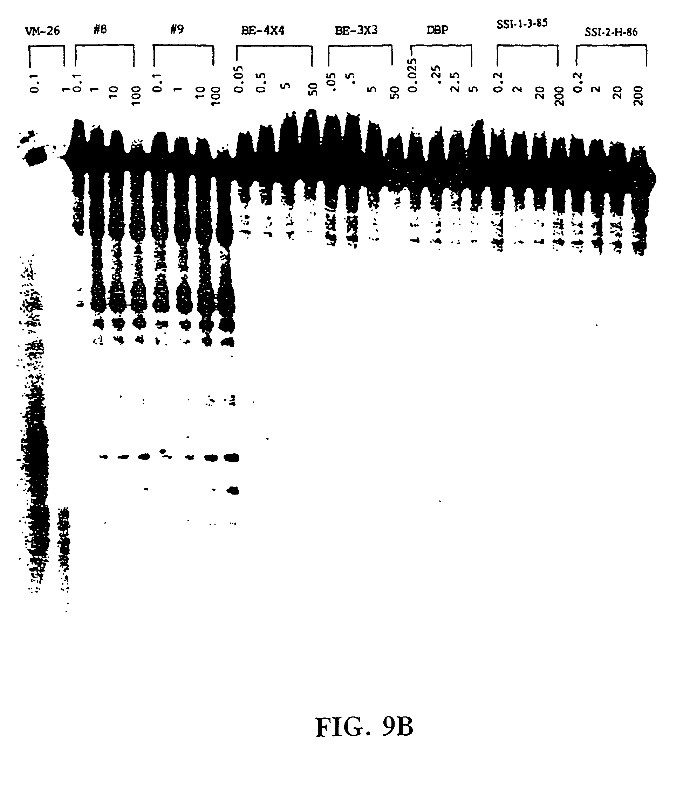

Analogous experiments to assay the ability of several of the compounds described herein to induce Topoisomerase II (Topo II)-mediated DNA cleavage were also performed. The results of one such experiment are depicted in the electrophoretegram of Figs. 9A and 9B. Here, DNA was incubated in the presence of Topo II and several different compounds described herein. The electrophoresis plate run depicted in Figs. 9A and 9B shows that the naphthoquinone derivatives described herein have the ability to form Topo II-drug DNA-cleaving complexes.

As detailed herein, the β-lapachone and dunnione analogs of the present invnetion have been shown to exhibit a broad spectrum of anti-cancer activity. The analogs are equally potent against human multidrug-resistant cancer cells. The gel depicted in Figs. 9A and 9B show that DNA Topo II is an intracellular target of the compounds described herein. The compounds stimulate Topo II-mediated DNA cleavage. The compounds also *induce* Topo II-mediated cleavage using purified mammalian Topo II. However, unlike other Topo II drugs, the DNA cleavage patterns induced by the naphthoquinone compounds were similar to background Topo II-induced cleavage.

The compounds described herein also inhibited Topo II catalytic activity in a P4 knotting assay (data not shown). The inhibition appears to be a specific interaction of the compounds with the Topo II-mediated reaction: the compounds induce very slight unwinding in a plasmid DNA unwinding assay. Furthermore, the compounds tested induced DNA cleavage and protein-DNA cross-links in cultured mammalian cells. This suggests that the anti-tumor activity of the compounds is due to a specific interaction with Topo II.

A legend for the compounds which were tested in Figs. 9A and 9B is as follows:

### Legend for Figs. 9A and 9B:

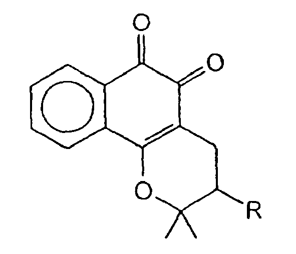

- Lane 1:: R = H (β-lapachone)
- Lane 2:: R = OH
- Lane 3:: R=OC(O)CH₃
- Lane 4:: R = CH₂CH=CH₂
- Lane 5:: R = OC(O)CH₂CH₂NH₃⁺Br
- Lane 6:: R = OC(O)CH₂CO₂H
- Lane 7:: 4-pentyloxy-1,2-naphthoquinone 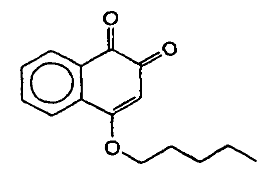
- Lane 8:: "Drug B," 3-NOR-dunnione
- Lane 9:: dunnione

The gels shown in Figs. 9A and 9B show that Topo II is an intracellular target of β-lapachone, dunnione, and their derivatives. In the far left-hand lane of Fig. 8A, DNA alone is shown. Moving to the right, the next lane contains DNA and Topo II (mammalian). Lanes 1-9 of Figs. 9A and 9B contain a series of concentrations of drugs (see Legend, above) in the presence of DNA and Topo II. Each set of lanes for each drug spans 3 orders of magnitude in concentration (0.1, 1, 10, and 100 µM). In Fig. 9A, the second lane from the left, which contains DNA and Topo II, shows very little DNA cleavage. The far left-hand lane of Fig. 9A, which contains DNA alone, shows no cleavage products. However, Lanes 1-9, which contain DNA, Topo II, and a drug according to the present invention, show extensive DNA cleavage. The wide range of differently-sized cleavage products indicates that the cleavage is extensive and heterogeneous.

### Biological Activity of 4-Substituted o-Naphthoquinones Against Neoplastic Cells

Examples 6-20 below, and the accompanying Figures, illustrate a series of experiments designed to illustrate the ability of the 4-substituted-1,2-*o-*naphthoquinones to induce cell death in several different neoplastic cell lines. Each graph has as its X-axis the concentration of the particular compound being tested. The Y-axis of each graph is a linear scale representing the fraction of cell survival in each of the cultures tested. The standardized testing protocol described above was used throughout all of the test cultures.

As specifically detailed in the Examples, the compounds of Formula III, wherein R is pentyloxy, 2-methyl-propyloxy, 3-methyl-butyloxy, isopropyloxy, 2-butenyloxy, benzyloxy, cyclohexylmethyloxy, 2-pentenyloxy, n-heptyloxy, n-pentylthio, and N,N-dimethylamino-ethylamino were shown to be highly effective in inhibiting cancer cell growth in MCF7 human breast cancer cells at concentrations well below 10 µM, and in some instances less than 5 µM.

Furthermore, the 4-substituted-1,2-napthoquinones of Formula III display equally lethal effects in several other cancer cell lines. The subject compounds display similar cytotoxic effects against human leukemias, including CEM, CEM/VM-1 (teniposide-resistant), and CEM/M70-B1 (merbarone-resistant) cell lines, as well as HL60 and HL60ADR (adriamycin-resistant) leukemia cell lines; human lung cancer cells including A549 cells, human colon cancer cells including HT29 cells (ATCC HTB-38), and human epidermoid cancer cells including KB3-1 (ATCC CCL-17), KBH-1, and its multi-drug resistant variant KB-VI (vinblastine-resistant). The compounds are also effective to inhibit the growth of non-human cancers, including the metastatic rat prostate cell line AT3.1.

### Biological Activity Against ADR-Resistant and MDR-Resistant Neoplastic Cells

It is well known that multiple-drug resistance (MDR) can be induced in neoplastic cells by anthracyclin quinones and quinoid-forming etoposides. In particular, the resistance of MCF7ADR cells is due to two distinct mechanisms. The first mechanism involves an ATP-driven efflux pump, and is known as the P-glycoprotein mechanism; the second mechanism involves an altered glutathione cycle.

KB-V1, a human epidermoid cancer cell line, is resistant to vinblastine, a known P-glycoprotein substrate. However, when exposed to the 4-substituted-1,2-naphthoquinones of Formula III, KB-V1 cells were killed to the same extent as were non-resistant KB-3-1 parent cells. This indicates that the subject compounds are not substrates for the P-glycoprotein efflux pump. Moreover, in a comptetive binding assay using ¹²⁵NAS-VP, a photoaffinity analog of verapamil, the binding of ¹²⁵NAS-VP to P-glycoprotein was not inhibited by up to 100 µM of the 4-substituted-1,2-naphthoquinones of Formula III. This lends support to the thought that the Formula III compounds are not exported from MDR cells via the P-glycoprotein efflux pump.

It does appear that the altered glutathione pathway of MDR cells plays a role in the cytotoxic effect of the subject compounds. When MCF7 and MCF7ADR cells were cultured in the presence of the Formula III compounds and L-butathione sulfoximine (BSO), a glutathione synthetase inhibitor, the sensitivity of MCF7ADR cells to the subject compounds increased by approximately three-fold. The cytotoxicity of the compounds against the MCF7 cells remained the same when treated with BSO. However, when treated with the subject compounds in the absence of BSO, the sensitivity of MCF7ADR cells was approximately the same as the sensitivity of MCF7 cells.

In short, treating ADR-resistant cells with a compound of Formula III revealed that there is no cross-resistance to the subject compounds.

The compounds of Formula III are also effective against cells which are resistant to topoisomerase II inhibitors. The human acute leukemia cell line CEM/VM-1 is resistant to teniposide (a topoisomerase-ATP dependent compound) and CEM/M70-B1 is resistant to merbarone (a topoisomerase II inhibitor). The subject 4-substituted-1,2-naphthoquinones were equally lethal to all CEM cells lines, including the parent type.

### Pharmaceutical Dosage Forms

The above-described compounds being effective to inhibit the growth of cancer cells, the compounds are suitable for the therapeutic treatment of neoplastic conditions in mammals, including humans. Cancer cell growth inhibition at pharmacologically-acceptable concentrations has been shown in human breast cancer, colon cancer, lung cancer, brain cancer, and prostate cancer cell lines, as described above.

The compounds described herein are administratable in the form of tablets, pills, powder mixtures, capsules, injectables, solutions, suppositories, emulsions, dispersions, food premixes, and in other suitable forms. The pharmaceutical dosage form which contains the compounds described herein is conveniently admixed with a non-toxic pharmaceutical organic carrier or a non-toxic pharmaceutical inorganic carrier. Typical pharmaceutically-acceptable carriers include, for example, mannitol, urea, dextrans, lactose, potato and maize starches, magnesium stearate, talc, vegetable oils, polyalkylene glycols, ethyl cellulose, poly(vinylpyrrolidone), calcium carbonate, ethyl oleate, isopropyl myristate, benzyl benzoate, sodium carbonate, gelatin, potassium carbonate, silicic acid, and other conventionally employed acceptable carriers. The pharmaceutical dosage form may also contain non-toxic auxillary substances such as emulsifying, preserving, or wetting agents, and the like.

Administration of the subject naphthoquinones to a human or non-human patient can be accomplished by any means known. The preferred administration route is parenteral, including intravenous administration, intraarterial administration, intratumor administration, intramuscular administration, intraperitoneal administration, and subcutaneous administration in combination with a pharmaceutical carrier suitable for the chosen administration route. The treatment method is also amenable to oral administration.

It must be noted, as with all pharmaceuticals, the concentration or amount of the naphthoquinone administered will vary depending upon the severity of the ailment being treated, the mode of administration, the condition and age of the subject being treated, and the particular naphthoquinone or combination of naphthoquinones being used.

Solid forms, such as tablets, capsules and powders, can be fabricated using conventional tabletting and capsule-filling machinery, which is well known in the art. Solid dosage forms may contain any number of additional non-active ingredients known to the art, including excipients, lubricants, dessicants, binders, colorants, disintegrating agents, dry flow modifiers, preservatives, and the like.

Liquid forms for ingestion can be formulated using known liquid carriers, including aqueous and non-aqueous carriers, suspensions, oil-in-water and/or water-in-oil emulsions, and the like. Liquid formulation may also contain any number of additional non-active ingredients, including colorants, fragrance, flavorings, viscosity modifiers, preservatives, stabilizers, and the like.

For parenteral administration, the subject compounds may be administered as injectable dosages of a solution or suspension of the compound in a physiologically-acceptable diluent or sterile liquid carrier such as water or oil, with or without additional surfactants or adjuvants. An illustrative list of carrier oils would include animal and vegetable oils (peanut oil, soy bean oil), petroleum-derived oils (mineral oil), and synthetic oils. In general, for injectable unit doses, water, saline, aqueous dextrose and related sugar solutions, and ethanol and glycol solutions such as propylene glycol or polyethylene glycol are preferred liquid carriers.

The pharmaceutical unit dosage chosen is preferably fabricated and administered to provide a concentration of drug at the point of contact with the cancer cell of from 1 µM to 10 mM. More preferred is a concentration of from 1 to 100 µM. This concentration will, of course, depend on the chosen route of administration and the mass of the subject being treated.

### EXAMPLES

The following Examples are provided solely to aid in a clear understanding of the presently claimed invention. The following Examples do not limit the scope of the invention described above or claimed herein in any fashion.

### Example 1: Preparation of C-allyl and O-allyl ether derivatives of lawsone

With reference to Reaction I, above, lawsone (2-hydroxy-1,4-naphthoquinone) (52.25 g, 300 mmol) was dissolved in anhydrous DMSO (350 mL) at 23°C. The solution was cooled to -78°C, and lithium hydride (2.50 g, 315 mmol) was added to the solid. The solid solution was then allowed to warm up slowly to 23°C. When gas evolution subsided, lithium iodide (10.0 g, 75 mmol) was added, followed by the allyl bromide (34.6 mL, 300 mmol), which was added dropwise. The mixture was stirred for 5 hours at 45°C and then for 10 hours at 23°C. After quenching the reaction with ice (200 g), water was added to the reaction (700 mL), followed by concentrated HCl (70 mL) and ethyl acetate (500 mL). Undissolved solids were collected by filtration and were confirmed to be the allyloxy-1,4-naphthoquinone (14, 20 g, 30%).
Dimethylallyloxy-1,4-naphthoquinone(14, R=R' =Me): ¹HNMR (CDCl₃. 300 MHz): δ 8.20-8.00 (m,2H), 7.86-7.58 (m, 2H), 6.16 (s, 1H), 4.49 (t, *J* = 6.8 Hz. 1H), 4.59 (d, *J* = 6.8 Hz, 2H), 1.81 (s, 3H), 1.76 (s, 3H).
2-Methylallyloxy-1,4-naphthoquinone (14, R=H, R'=Me): mp 136.0-137.0 °C, ¹H NMR (CDCl₃, 300 MHz) δ 8.13 (dd, *J* = 7,2 Hz, 1H), 8.08 (dd, *J* =7, 2 Hz, 1H), 7.75 (dt, *J* = 7, 2 Hz, 1H), 7.70 (dd, *J* = 7, 2 Hz, 1H), 6.17 (s, 1H), 6.00-5.90 (m, 1H), 5.8-5.7 (M, 1H), 4.53 (d, *J* = 6 Hz, 2H), 1.78 (d, *J* = 6 Hz, 3H).
2-Allyloxy-1,4-naphthoquinone(14. R=R' =H): ¹H NMR (CDCl₃, 300 MHz) δ 8.22-8.04 (m, 2H), 7.94-7.66 (m, 2H), 6.17 (s, 1H), 6.15-5.95 (m, 1H), 5.49 (dd, *J* = 11.6, 1.2 Hz, 1H), 5.41 (dd, *J* = 10.5, 1.2 Hz, 1H), 4.62 (d, *J* = 5.6 Hz, 2H).

The ethyl acetate layer was then separated and aqueous layer extracted again with more ethyl acetate (250 mL). The combined organic layers were then extracted with 5 % aqueous NaHCO₃. The NaHCO₃ extracts were acidified with concentrated HCl and the precipitate filtered. The precipitate was shown to be unreacted lawsone (10) (16.02 g, 30%).

The ethyl acetate solution was evaporated *in vacuo* and the residue dissolved in diethyl ether (500 mL). The ether solution was extracted with 2 N NaOH (3 X 200 mL). The alkaline extracts were acidified with concentrated HCl and allowed to stand at 4°C for 15 hours. The precipitate was filtered, dried, and re-crystallized from a mixture of EtOH/H₂O to afford (13) as yellow crystals.
3-(Dimethylallyl)-2-hydroxy-1,4-naphthoquinone(13, R=R'=Me) (28.76 g, 40%): ¹H NMR (CDCl₃, 300 MHz) δ 8.12 (d, *J* = 7.5 Hz, 1H), 8.07 (d, *J* = 7.5, 1H), 7.75 (t, *J* = 7.5 Hz, 1H), 7.67 (t, *J* = 7.5 Hz, 1H), 7.29 (s, OH), 5.21 (t, *J* = 7.3 Hz, 1H), 3.31 (d, *J* = 7.3 Hz, 1H), 1.79 (s, 3H), 1.68 (s, 3H).
3-(Methylallyl)-2-hydroxy-1,4-naphthoquinone (13, R=H, R'=Me) (36%): mp 130.5-131.5°C; ¹H NMR (CDCl₃, 300 MHz) δ 8.13 (dd, *J* = 7.5, 1.3 Hz, 1H), 8.08 (dd, *J* = 7.5, 1.3 Hz, 1H), 7.76 (td, *J* =7.5, 1.3 Hz, 1H), 7.68 (td, *J* = 7.5, 1.3 Hz, 1H), 7.36 (s, OH), 5.40-5.70 (m, 2H), 3.29 (d, *J* = 6.0 Hz, 2H), 1.62 (d, *J* = 6.0 Hz, 2H).
3-Allyl-2-hydroxy-1,4-naphthoquinone (13, R=R'=H) (39%): ¹H NMR (CDCl₃, 300 MHz) δ 8.13 (dd, *J* = 7.7, 1.1 Hz, 1H), 8.09 (dd, *J* = 7.6, 1.1 Hz, 1H), 7.76 (dt, *J* = 7.5, 1.5 Hz, 1H), 7.69 (dt, *J* = 7.5, 1.4 Hz, 1H), 7.33 (s, OH), 6.08-5.80 (M, 1H), 5.17 (dd, *J* = 17.1, 1.6 Hz, 1H), 5.05 (dd, *J* = 10.0, 1.5 Hz, 1H), 3.37 (dt, *J* = 6.5, 1.4 Hz, 2H).

### Example 2: The Claisen rearrangement

Referring now to Reaction III, above, a solution of (14) (20 g) in toluene (250 mL) was heated to reflux. The solids dissolved gradually as the temperature increased and a clear red-pink solution resulted. After heating under reflux for 1.5 h, the solution was allowed to cool and 2 N NaOH (100 mL) was added. The solution was filtered to separate unreacted ally ether (14) (2.24 g, 11%). The aqueous layer was separated and the toluene layer was extracted with more 2 N NaOH (2 X 50 mL). The combined aqueous layers were acidified with concentrated HCl and extracted with ethyl acetate. The organic extracts were dried, concentrated *in vacuo,* and the residue re-crystallized from EtOH/H₂O to afford (17).
3-(Dimethylallyl)-2-hydroxy-1,4-naphthoquinone (17, R=R' =Me) (12.67 g, 63%): ¹H NMR (CDCl₃, 300 MHz) δ 8.10-8.00 (m, 2H), 7.84 (s, OH), 7.80-7.60 (m, 2H), 6.29 (dd, *J* = 17.5, 10.6 Hz, 1H), 5.04-4.93 (m, 2H), 1.57 (s, 6H).
3-(Methylallyl)-2-hydroxy-1,4-naphthoquinone (17, R=H, R'=Me) (30%): ¹H NMR (CDCl₃, 300 MHz) δ 8.12 (dd, *J* = 7.7, 1.1 Hz, 1H), 8.07 (dd, *J* = 7.6, 1.2 Hz, 1H), 7.76 (dt, *J* = 7.5, 0.9 Hz, 1H), 7.68 (dt, *J* = 7.5, 1.3 Hz, 1H), 6.20 (ddd, *J* = 17.3, 10.1. 7.4 Hz, 1H), 5.14 (dd, *J* = 17.1, 1.5 Hz, 1H), 5.02 (dd, *J* = 10.1, 1.3 Hz, 1H), 3.99 (m, 1H), 1.41 (d, *J* = 7.1 Hz, 3H).

### Example 3: Preparation of β-lapachone and analogs

### Cyclization via treatment with strong acid:

One technique to form the third ring of the tricyclic compounds is to treat the allyl intermediate with concentrated acid. With reference to Reactions II and III, concentrated sulfuric acid (70 mL) was added to lapachol (13) (11.26 g) (or 17 in Reaction III) at 23°C. After stirring until all solids dissolved (approximately 15 minutes), the mixture was poured into water (200 mL) and filtered to afford β-lapachone (15, R5 =R6=Me) (11.11 g, 99%).
Re-crystallization from diethyl ether gave β-lapachone as orange needles (10.45 g, 94% recovery): mp 154-155.5°C, ¹H NMR (CDCl₃, 300 MHz) δ 8.06 (d, *J* = 7.7 Hz, 1H), 7.81 (d, *J* = 7.7 Hz, 1H), 7.64 (t, *J* = 7.7 Hz, 1H), 7.50 (t, *J* = 7.5 Hz, 1H), 2.58 (t, *J* = 6.7 Hz, 2H), 1.86 (t, *J* = 6.7 Hz, 2H), 1.47 (s, 6H).
Monomethyl-β-lapachone(15, R=H, R'=Me) (48% along with 22% α-isomer after silica gel chromatography): mp 164-165°C; ¹H NMR (CDCl₃, 300 MHz) δ 8.07 (dd, *J* = 7.6, 1.5 Hz. 1H), 7.82 (dd, *J* = 7.6, 1.2 Hz, 1H), 7.65 (dd, *J* = 7.6, 1.5 Hz, 1H), 7.51 (dd, *J* = 7.6, 1.2 Hz, 1H), 4.40 (dqd, *J* = 10, 6.3, 3 Hz, 1H), 2,71 (ddd, *J* = 17.5, 5.5, 3.5 Hz, 1H), 2.46 (ddd, *J* = 17.5, 10.7, 6.0 Hz, 1H), 2.11 (dddd, *J* = 14, 6, 3.5, 3, 1H), 1.71 (dddd, *J* = 14, 10.7, 10, 5.5 Hz, 1H), 1.54 (d, *J* = 6.3 HZ, 3H).
Dunnione (16, R=R' =Me): ¹H NMR (CDCl₃, 300 MHz) δ 8.04 (d, *J* = 7.5 Hz, 1H), 7.68-7.50 (m, 3H), 4.67 (q, *J* = 6.7 Hz, 1H), 1.47 (d, *J* = 6.7 Hz, 3H), 1.45 (s, 3H), 1.27 (s, 3H).
3-NOR-dunnione (16, R=H, R'=Me, R2 and R4=H) (30%): ¹H NMR (CDCl₃, 300 MHz) δ 8.08 (d, *J* = 7.4 Hz, 1H), 7.72-7.50 (m, 3H), 5.24-5.12 (m, 1H), 3.60-3.48 (m, 1H), 1.54 (d, *J* = 6.7 Hz, 3H), 1.24 (d, *J* = 7.1 Hz, 3H).

### Cyclization via epoxidation to yield 3-substituted lapachones:

Referring now to Reaction IV, 3-hydroxy-β-lapachone (19) and derivatives thereof can be synthesized by forming an epoxide intermediate followed by ring closure. An illustrative synthesis of 3-hydroxy-β-lapachone (19, R5=R6=Me, R7=OH) proceeds as follows:

Lapachol (12.11 g, 50 mmol) was dissolved in CH₂Cl₂ (250 mL) at 23°C. The solution was cooled to 0°C, which caused some lapachol to precipitate. To this cooled solution was added m-chloroperoxybenzoic acid (m-CPBA) (10.15 g, 85 % purity, 50 mmol). The solution then was stirred for 4 hours at 23°C, and the solution filtered. The filtrate was washed with aqueous NaHCO₃ and dried.

The epoxide so formed (18) remains in solution. To this solution was added BF₃•OEt₂ (6.15 mL, 50 mmol) at 0°C. After stirring at 23°C for 10 hours, the solution was washed consecutively with aqueous Na₂CO₃, 5% citric acid, and water. The organic layer was extracted with 5% NaHSO₃ (300 mL, 200 mL, 200 mL). The extracts were pooled. Saturated Na₂CO₃ (600 mL) was added to the pooled extracts to yield reddish precipitates. The solution containing the precipitates was cooled at 0°C for 2 hours and filtered.
The filtrate is 3-hydroxy-β-lapachone (19, R=R' =Me, R" =H) (6.72 g, 52 % for the two steps from lapachol): mp 202.5-203.5°C, ¹H NMR (CDCl₃, 300 MHz) δ 8.06 (d, *J* = 7.6 Hz, 1H), 7.84 (d, *J* = 7.6 Hz, 1H), 7.66 (t, *J* = 7.6 Hz, 1H), 7.52 (t, *J* = 7.6 Hz, 1H), 3.92 (m, 1H), 2.83 (dd, *J* = 17.7, 4.8 Hz, 1H), 2.62 (dd, *J* = 17.7, 5.4 Hz, 1H), 1.52 (s, 3H), 1.46 (s, 3H).
2-Hydroxy-3-(2',3'-oxo-3'methylbutyl)naphthoquinone(18). This compound can be obtained by repeating the reaction described immediately above, and isolating the epoxide by silica gel chromatography (25-100% ethyl acetate in hexanes). ¹H NMR (DMSO-d6, 300 MHz) δ 8.04-7.95 (m, 2H), 7.87-7.72 (m, 2H), 3.52 (t, *J* = 6.5 Hz, 1H), 2.65 (d, *J* = 6.8 Hz, 2H), 1.10 (s, 6H); ¹³C NMR (CDCl₃, 75 MHz) δ 180.92 (s), 175.13 (s), 169.94 (s), 134.53 (d) < 131.88 (d), 130.43 (s), 129.23 (d), 127.21 (s), 124.50 (d), 115.94 (s), 93.59 (d), 71.52 (s), 27.26 (t), 25.62 (q), 24.56 (q).

### Example 4: Fully-Aromatic Dunnione Analogs

With reference to Reaction V, above, the fully-aromatic derivative of dunnione, namely 2-methyl-4H,5H-naphtho(2,3-b)furan-6,7-dione (21), can be synthesized by reacting lawsone with an aldehyde.

Illustratively, 2-hydroxy-3-propenyl-1,4-naphthoquinone(20) was synthesized, followed by ring closure to yield the fully-aromatic dunnione derivative, as follows:

Propionaldehyde (RCHO, R=propyl, 5.0 mL, 69.3 mmol) was added to a solution of concentrated HCl (2 mL) and lawsone (2.00 g, 11.5 mmol) in acetic acid (35 mL) at 60°C. After stirring for 1.25 hours, another portion of propionaldehyde was added (5.0 mL, 69.3 mmol). The solution was then stirred for an additional 1 hour. The solution was allowed to cool to room temperature, and then ice water (200 mL) was added to quench the reaction. The solution was extracted with diethyl ether (3 x 200 mL) and the organic fractions pooled. The combined organic layers were re-extracted with 5% Na₂CO₃ (8 x 150 mL). The aqueous extracts were also pooled and acidified with concentrated HCl. A precipitate formed which was collected by filtration to afford (20) as an orange solid. 2-Hydroxy-3-propenyl-1,4-naphthoquinone (20) (983 mg, 40%): mp 133-134°C; ¹H NMR (CDCl₃, 300 MHZ) δ 8.13 (d, *J* = 7.7 Hz, 1H), 8.06 (d, *J* = 7.5 Hz, 1H), 7.80-7.62 (m, 3H), 7.15-6.95 (m, 1H), 6.63 (d, *J* = 16.1 Hz, 1H), 1.99 (d, *J* = 6.8 Hz, 3H).

Ring closure to yield 2-methyl-4H, 5H-naphtho[2,3-b]furan-6,7-dione(21) can be accomplished as follows. A solution of the naphthoquinone (20) (2.26 g) and Hg(OAc)₂ (5.0 g) in acetic acid (200mL) was stirred for 10 hours at 23°C. The precipitate formed which was removed by filtration. The filtrate was poured into water (400 mL), and the resultant solution was extracted with ethyl acetate (3 x 200 mL). The combined extracts were washed with water (3 x 200 mL). After drying over MgSO₄, the organic layer was concentrated *in vacuo,* and the residue was purified by chromatography (10-20% ethyl acetate in hexane) on silica gel to give (21) as a brown-red solid.
2-Methyl-4H, 5H-naphtho(2,3-b)furan-6,7-dione (21, (3,3-DINOR-2,3-dehydrodunnione)): (549 mg, 25%) mp 158.5-160°; ¹H NMR (CDCl₃, 300 MHz) δ 8.05 (d, *J* = 7.6 Hz, 1H), 7.68-7.57 (m, 2H), 7.43 (dt, *J* = 7.3, 1.8 Hz, 1H), 6.45 (s, 1H), 2.43 (s, 3H); ¹³C NMR (CDCl₃, 75 MHz) δ 180.73 (s), 174.40 (s), 159.64 (s), 155.97 (s), 135.39 (d), 130.47 (d), 129.82 (d), 128.72 (s), 128.60 (s), 122.70 (s), 121.99 (d), 104.55 (d), 13.63 (q); MS *m*/*z* 69, 128, 183, 212; HRMS *m*/*z* calculated for C₁₃H₈O₃ (M+) 212.0473, found 212.0471.

### Example 5: Derivatization of 3-hydroxy-β-lapachone

Once 3-hydroxy-β-lapachone has been isolated, its hydroxyl functionality can be utilized to synthesize a wide range of 3-oxy-substituted β-lapachone derivatives. What follows are examples of a mono-acid derivative, an amino acid derivative, and a di-acid derivative. Based upon these illustrative syntheses, several analogous derivatives can be synthesized with ease.

With reference to Reaction VI, above, 1,1'-carbonyldiimidazole (486 mg, 3.0 mmol) was first added to the corresponding carboxylic acid (3.0 mmol) in dimethylformamide (DMF) (8 mL) at 23°C. For the following examples only, R¹⁰ of the acid and corresponding 3-substituted lapachone product can be CH₂CH₂NHBoc-(Boc = *t*-butoxycarbonyl) (22a), CH₂CO₂C(CH₃)₂- (22b), or methyl (22c).

After stirring for 20 minutes, 3-hydroxy-β-lapachone (19) (517 mg, 2.0 mmol) and DBU (389 uL, 26 mmol) were added to the mixture. (DBU = 1,8-diazabicyclo(5.4.0)undec-7-ene, a relatively strong, sterically-hindered, non-nucleophilic base.) The mixture was stirred for 5 hours and poured into water (150 mL). The precipitate was collected by filtration and purified by silica gel chromatography (10-33% ethyl acetate in hexanes) to afford (22) (approximately 50 % yield).

With reference to Reaction VII, compound (22a) (200 mg, 0.466 mmol) was added to diethyl ether (200 mL). A small amount of undissolved residue was removed by filtration. To the clear solution, hydrogen bromide (35% in acetic acid, 3.0 mL) was added at 23°C. After stirring for 10 minutes, the solution was filtered, and the precipitate re-crystallized from methanol to afford (23) (52 mg, 27%): ¹H NMR (D₂O, 300 MHz) δ 8.05-7.60 (m, 4H), 5.13 (m, 1H), 3.10 (t, *J* = 6.5 Hz, 2H), 2.84-2.50 (M, 4H), 1.39 (s, 3H), 1.31 (s, 3H).

Referring now to Reaction VIII, trifluoracetic acid (1.0 mL) was added to a solution of compound (22b) (330 mg) in CH₂Cl₂ (1.5 mL) at 23°C. After stirring I hour the mixture was concentrated *in vacuo.* The residue was dissolved in MeO-*t*-Bu (50 mL) and extracted with saturated NaHCO₃ (2 X 25 mL). The combined aqueous layers were counter-extracted with diethyl ether and acidified with concentrated HCl. The resultant precipitate was filtered to give the malonyl derivative (24) (136 mg, 48%): ¹H NMR (CDCl₃, 300 MHz) δ 8.08 (d, *J* = 7.6 Hz, 1H), 7.85 (d, *J* = 7.8 Hz, 1H), 7.69 (t, *J* = 7.6 Hz, 1H), 7.55 (t, *J* = 7.5 Hz, 1H), 5.19 (t, *J* = 4.6 Hz, 1H), 3.36 (s, 2H), 2.84 (dd, *J* = 18.2, 4.9 Hz, 1H), 2.73 (dd, *J* = 18.2, 4.4 Hz, 1H), 1.52 (s, 3H), 1.48 (s, 3H); ¹³C NMR (CDCl₃, 75 MHz) δ 179.43 (s), 178.64 (s), 170.12 (s), 161.22 (s), 134.98 (d), 132.07 (s), 131.08 (d), 130.21 (s), 128.93 (d), 124.37 (d), 110.12 (s), 79.76 (s), 69.11 (d), 24.98 (q), 23.29 (q), 22.71 (t), 21.02 (q); MS (FAB) *m*/*z* 136, 154, 241, 345 (MH⁺).
3-(*n*-Butyloxycarboxy-β-alanyloxy)-β-lapachone (22a):¹H NMR (CDCl₃, 300 MHz) δ 8.10 (d, 1H), 7.84 (d, 1H), 7.67 (t, 1H), 7.54 (t, 1H), 5.17 (t, 1H), 3.53-3.32 (m, 1H), 2.93 (br, 1H), 2.85 (dd, 1H), 2.70 (dd, 1H), 2.65-2.50 (m, 1H), 1.47 (d, 6H), 1.42 (s, 9H).
3-(β-Alanyloxy)-β-lapachone (23): mp 228-229°C (decomposed); ¹H NMR (D₂O, 300 MHz) δ 8.05-7.60 (m, 4H), 5.13 (m, 1H), 3.10 (t, *J* = 6.5 Hz, 2H), 2.84-2.50 (m, 4H), 1.39 (s, 3H); ¹³C NMR (D₂O, 75 MHz, DMSO-d6 was added as internal standard) δ 181.77 (s), 180.84 (s), 173.02 (s), 165.13 (s), 137.45 (d), 132.93 (s), 130.79 (s), 130.03 (d), 126.34 (d), 110.92 (s), 82.29 (s), 71.99 (d), 36.34 (t), 32.57 (t), 25.41 (q), 24.10 (q), 23.35 (t); MS (FAB) *m*/*z* 122.0, 205.1, 241.1, 301.1, 330.1, (MH⁺), 659.1 (2M+H⁺).
3-(2'-*t*-Butyloxycarboxyacetoxy)-β-lapachone(22b) (27%): ¹H NMR (CDCl₃, 300 MHz) δ 8.10 (d, 1H), 7.84 (d, 1H), 7.68 (t, 1H), 7.55 (t, 1H), 5.22 (t, 1H), 3.33 (s, 2H), 2.95 (dd, 1H), 2.74 (dd, 1H), 1.52 (d, 6H), 1.42 (s, 9H).
3-Acetoxy-β-lapachone (22c): ¹H NMR (CDCl₃. 300 MHz) δ 8.09 (d, *J* = 7.6 Hz, 1H), 7.85 (d, *J* = 7.8 Hz, 1H), 7.68 (t, *J* = 7.6 Hz, 1H), 7.55 (t, *J* = 7.6 Hz, 1H), 5.15 (t, *J* = 4.5 Hz, 1H), 2.82 (dd, *J* = 18.2, 4.8 Hz, 1H), 2.68 (dd, *J* = 18.2, 4.1 Hz, 1H), 2.08 (s, 3H), 1.49 (s, 3H), 1.44 (s, 3H); ¹³CNMR (CDCl₃, 75 MHz) δ 179.43 (s), 178.64 (s), 170.12 (s), 161.22 (s), 134.98 (d), 132.07 (s), 131.08 (d), 130.21 (s), 128.93 (d), 124.37 (d), 110.12 (s), 79.76 (s), 69.11 (d), 24.98 (q), 23.29 (q), 22.71 (t), 21.02 (q).

### Example 6: 4-Pentyloxy-1,2-napthoquinone Against MCF7 Cells and Adriamycin-Resistant MCF7 Cells

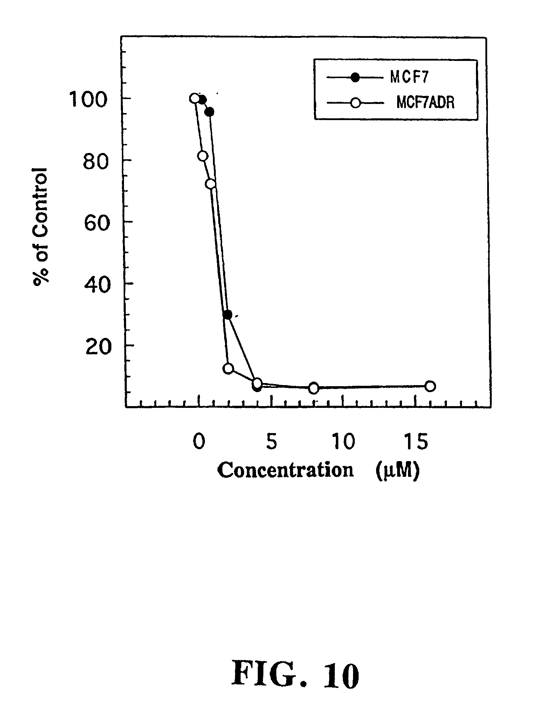

Following the standard protocol, the survival of cultured human breast cancer cells MCF7 and adriamycin-resistant MCF7 cells in the presence of increasing concentrations of 4-pentyloxy-1,2-napthoquinone was investigated. The results of both studies are depicted in Fig. 10. The plots for the survival of cultured MCF7 breast cancer cells (●) and adriamycin-resistant MCF7 breast cancer cells (MCF7ADR) (○) as shown in Fig. 10 are virtually identical. At a concentration of less than 5 µM, 4-pentyloxy-1,2-napthoquinone effectively and equally inhibits the growth of both MCF7 and MCF7ADR cells in culture.

### Example 7: 4-Pentyloxy-1,2-napthoquinone Against MCF7 Cells, Adriamycin-Resistant MCF7 Cells, and MCF7 Cells Expressing High Levels of Glutathione Peroxidase

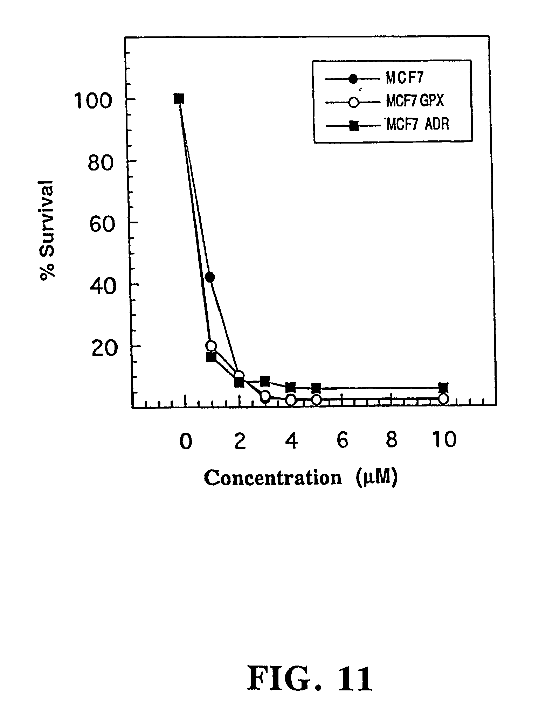

Following the standard protocol, the survival of cultured human breast cancer cells MCF7, adriamycin-resistant MCF7 cells, and MCF7 breast cancer cells containing a high level of cDNA-expressed glutathione peroxidase (MCF7GPX), in the presence of increasing concentrations of 4-pentyloxy-1,2-napthoquinone was investigated. The results of all three studies are depicted in Fig. 11. As in Example 6, the plots for the survival of cultured MCF7 breast cancer cells (●), adriamycin-resistant MCF7 breast cancer cells (MCF7ADR) (■), and MCF7 breast cancer cells containing a high level of cDNA-expressed glutathione peroxidase (MCF7GPX) (○) as shown in Fig. 11 are virtually identical. At concentrations of approximately 3 µM, 4-pentyloxy-1,2-napthoquinone effectively and equally inhibits the growth of MCF7, MCF7ADR, and MCF7GPX cells in culture.

### Example 8: 4-Pentyloxy-1,2-napthoguinone Against CEM Leukemia Cells, Teniposide-Resistant CEM Leukemia and Merbarone-Resistant Leukemia

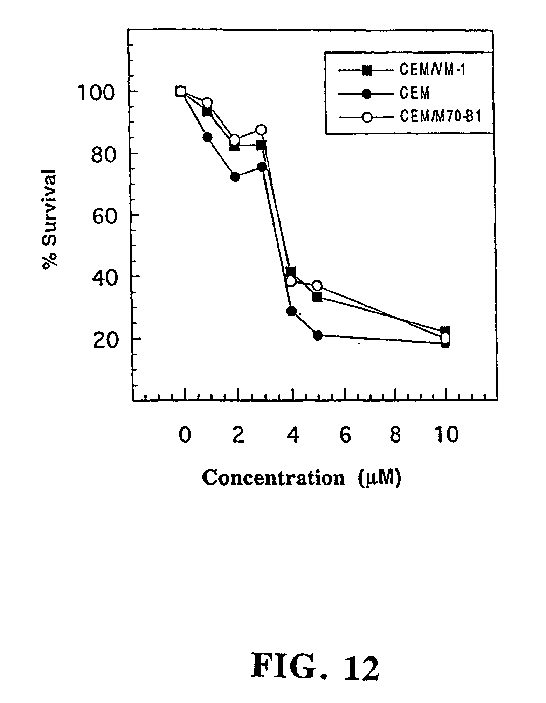

Following the standard protocol, the survival of cultured CEM leukemia cells, teniposide-resistant CEM leukemia cells (CEM-VM-1), and merbarone-resistant leukemia cells (CEM/M70-B1) (○) in the presence of increasing concentrations of 4-pentyloxy-1,2-napthoquinone was investigated. The results of the three studies are depicted in Fig. 12. As in the previous two Examples, the plots for the survival of cultured CEM leukemia cells (●), teniposide-resistant CEM leukemia cells (CEM-VM-1) (■), and merbarone-resistant leukemia cells (CEM/M70-B1) (○) are virtually identical and show effective inhibition of cell growth in both CEM cells and drug-resistant CEM cells in culture.

### Example 9: 4-(2-Methyl-propyloxy)-1,2-napthoquinoneAgainstMCF7 Cells and Adriamycin-Resistant MCF7 Cells

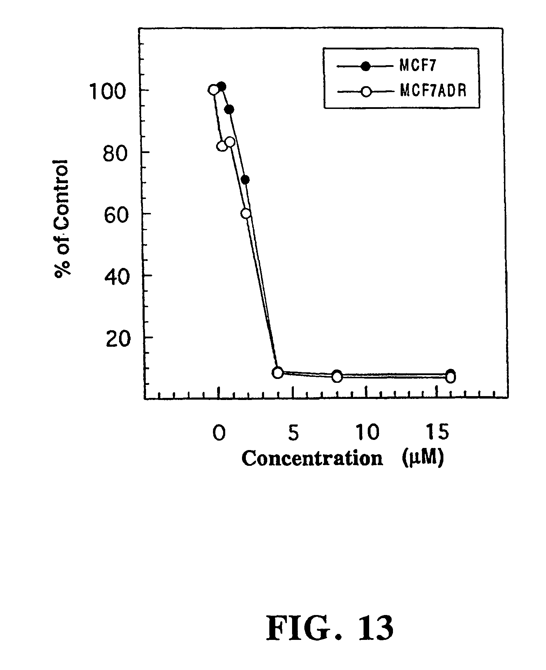

Following the standard protocol, the survival of cultured human breast cancer cells MCF7 and adriamycin-resistant MCF7 cells in the presence of increasing concentrations of 4-(2-methyl-propyloxy)-1 ,2-napthoquinone was investigated. The results of both studies are depicted in Fig. 13. The plots for the survival of cultured MCF7 breast cancer cells (●) and adriamycin-resistant MCF7 breast cancer cells (MCF7ADR) (○) are identical. At a concentration of less than 5 µM, 4-(2-methyl-propyloxy)-1,2-napthoquinone effectively and equally inhibits the growth of both MCF7 and MCF7ADR cells in culture.

### Example 10: 4-(3-Methyl-butyloxy)-1,2-napthoquinoneAgainst MCF7 Cells and Adriamycin-Resistant MCF7 Cells

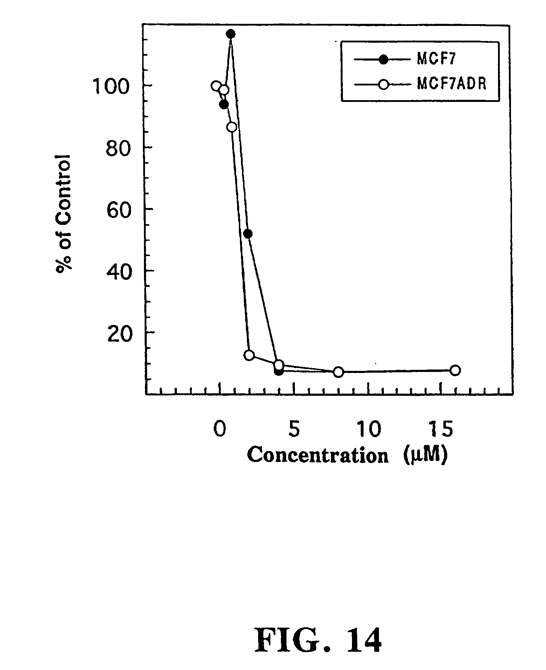

Following the standard protocol, the survival of cultured human breast cancer cells MCF7 and adriamycin-resistant MCF7 cells in the presence of increasing concentrations of 4-(3-methyl-butyloxy)-1,2-napthoquinone was investigated. The results of both studies are depicted in Fig. 14. The plots depict the survival of cultured MCF7 breast cancer cells (●) and adriamycin-resistant MCF7 breast cancer cells (MCF7ADR) (○) in the presence of 4-(3-methyl-butyloxy)-1,2-napthoquinone. At a concentration of less than 5 µM, 4-(3-methyl-butyloxy)-1,2-napthoquinone effectively and equally inhibits the growth of both MCF7 and MCF7ADR cells in culture.

### Example 11: 4-(Isopropyloxy)-1,2-napthoquinone Against MCF7 Cells and Adriamycin-Resistant MCF7 Cells

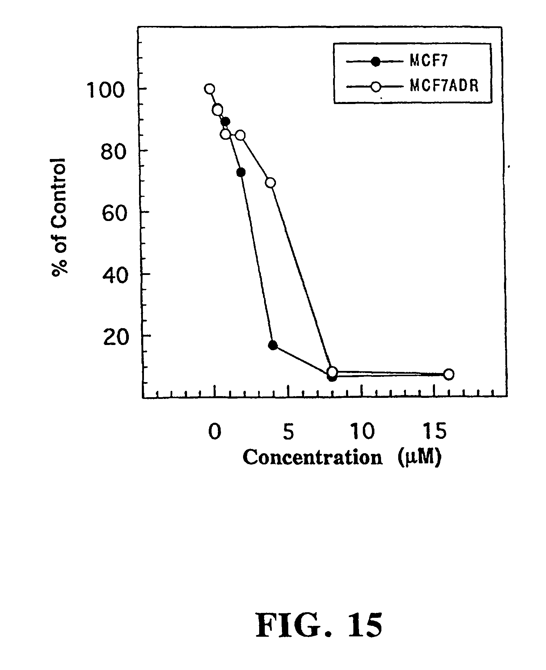

Following the standard protocol, the survival of cultured human breast cancer cells MCF7 and adriamycin-resistant MCF7 cells in the presence of increasing concentrations of 4-(isopropyloxy)-1,2-napthoquinone was investigated. The results of both studies are depicted in Fig. 15. The plots depict the survival of cultured MCF7 breast cancer cells (●) and adriamycin-resistant MCF7 breast cancer cells (MCF7ADR) (○). At a concentration of about 8 µM, 4-(isopropyloxy)-1,2-napthoquinone effectively and equally inhibits the growth of both MCF7 and MCF7ADR cells in culture.

### Example 12: 4-(2-Butenyloxy)-1,2-napthoquinone Against MCF7 Cells and Adriamycin-Resistant MCF7 Cells

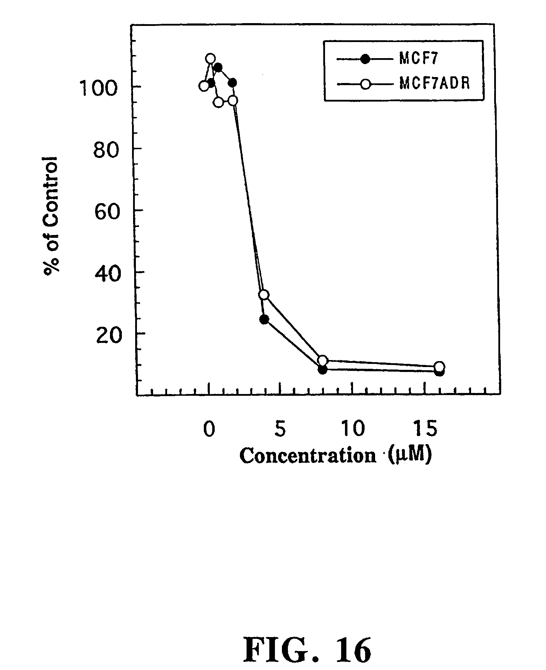

Following the standard protocol, the survival of cultured human breast cancer cells MCF7 and adriamycin-resistant MCF7 cells in the presence of increasing concentrations of 4-(2-butenyloxy)-1,2-napthoquinone was investigated. The results of both studies are depicted in Fig. 16. The plots depict the survival of cultured MCF7 breast cancer cells (●) and adriamycin-resistant MCF7 breast cancer cells. (MCF7ADR) (○). At a concentration of about 8 µM, 4-(2-butenyloxy)-1,2-napthoquinone effectively and equally inhibits the growth of both MCF7 and MCF7ADR cells in culture.

### Example 13: 4-Benzyloxy-1,2-napthoquinone Against MCF7 Cells and Adriamycin-Resistant MCF7 Cells

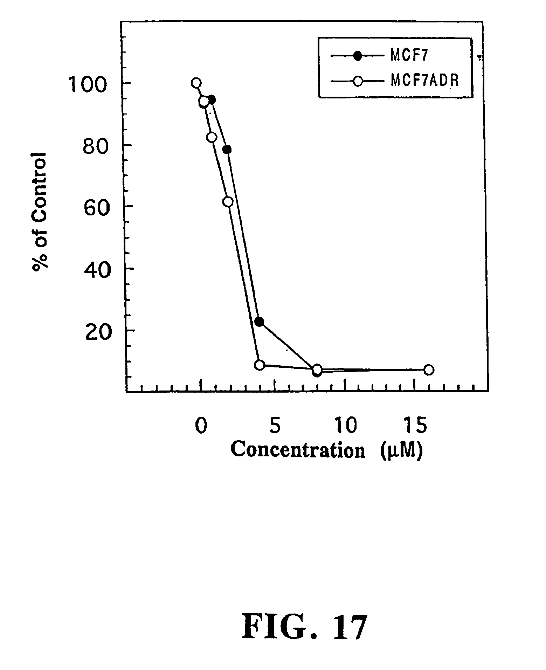

Following the standard protocol, the survival of cultured human breast cancer cells MCF7 and adriamycin-resistant MCF7 cells in the presence of increasing concentrations of 4-benzyloxy-1,2-napthoquinone was investigated. The results of both studies are depicted in Fig. 17. The plots depict the survival of cultured MCF7 breast cancer cells (●) and adriamycin-resistant MCF7 breast cancer cells (MCF7ADR) (○).

### Example 14: 4-Cyclohexylmethyloxy-1,2-napthoquinoneAgainstMCF7 Cells and Adriamycin-Resistant MCF7 Cells

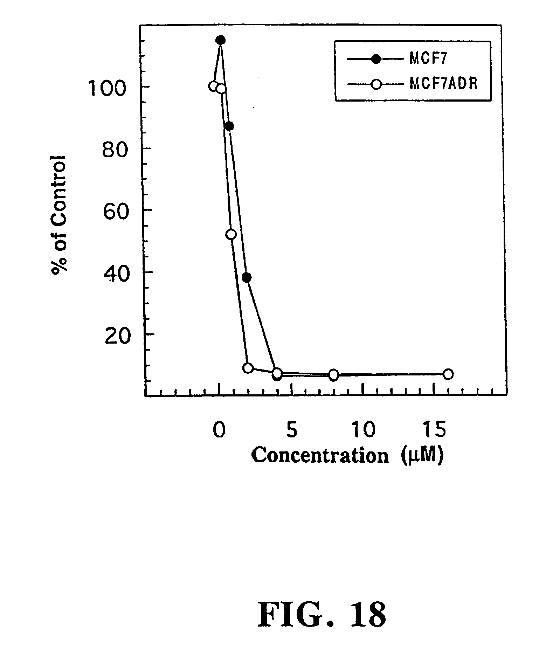

Following the standard protocol, the survival of cultured human breast cancer cells MCF7 and adriamycin-resistant MCF7 cells in the presence of increasing concentrations of 4-cyclohexylmethyloxy-1,2-napthoquinone was investigated. The results of both studies are depicted in Fig. 18. The plots depict the survival of cultured MCF7 breast cancer cells (●) and adriamycin-resistant MCF7 breast cancer cells (MCF7ADR) (○). At a concentration less than 5 µM, 4-cyclohexylmethyloxy-1,2-napthoquinone effectively and equally inhibits the growth of both MCF7 and MCF7ADR cells in culture.

### Example 15: 4-(2-Pentenyloxy)-1,2-napthoquinone Against MCF7 Cells and Adriamycin-Resistant MCF7 Cells

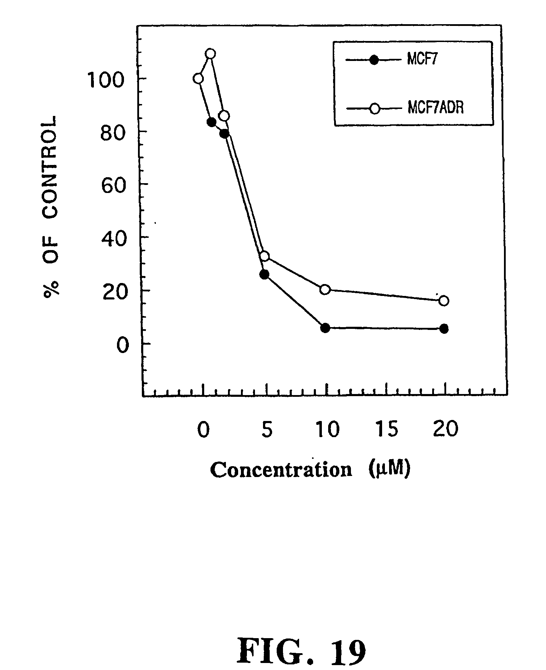

Following the standard protocol, the survival of cultured human breast cancer cells MCF7 and adriamycin-resistant MCF7 cells in the presence of increasing concentrations of 4-(2-pentenyloxy)-1,2-napthoquinone was investigated. The results of both studies are depicted in Fig. 19. The plots depict the survival of cultured MCF7 breast cancer cells (●) and adriamycin-resistant MCF7 breast cancer cells (MCF7ADR) (○).

### Example 16: 4-(n-Heptyloxy)-1,2-napthoquinone Against MCF7 Cells and Adriamycin-Resistant MCF7 Cells

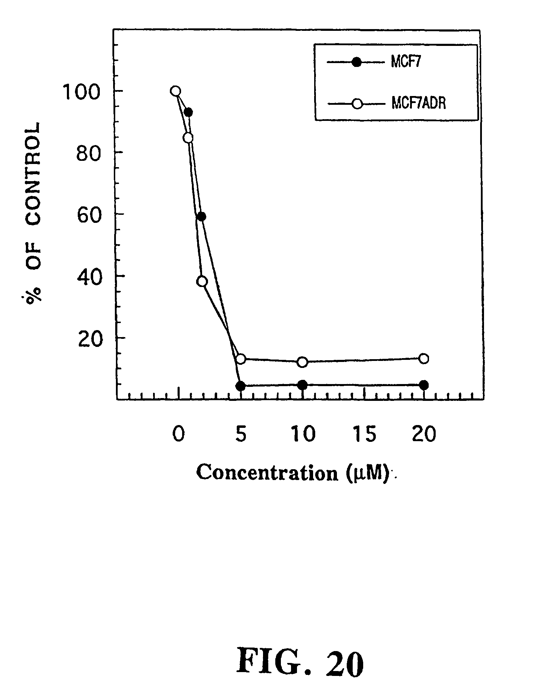

Following the standard protocol, the survival of cultured human breast cancer cells MCF7 and adriamycin-resistant MCF7 cells in the presence of increasing concentrations of 4-(n-heptyloxy)-1,2-napthoquinone was investigated. The results of both studies are depicted in Fig. 20. The plots depict the survival of cultured MCF7 breast cancer cells (●) and adriamycin-resistant MCF7 breast cancer cells (MCF7ADR) (○). At a concentration about 5 µM, 4-(n-heptyloxy)-1,2-napthoquinone effectively inhibits the growth of both MCF7 and MCF7ADR cells in culture.

### Example 17: 4-(n-Pentylthio)-1,2-napthoquinone Against MCF7 Cells and Adriamycin-Resistant MCF7 Cells

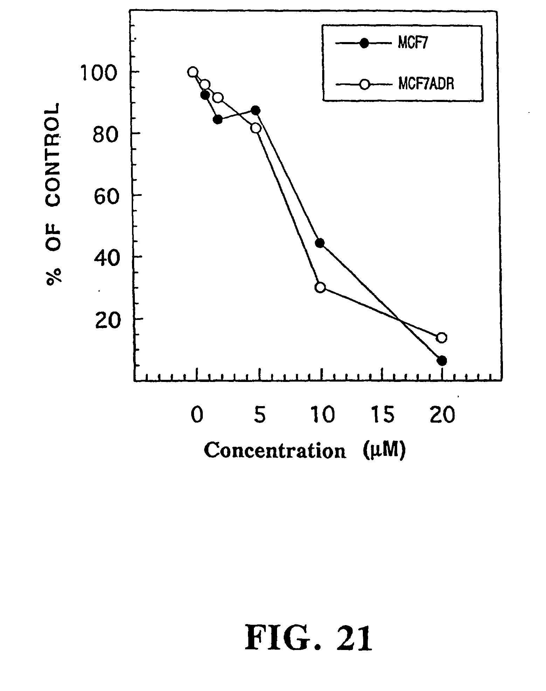

Following the standard protocol, the survival of cultured human breast cancer cells MCF7 and adriamycin-resistant MCF7 cells in the presence of increasing concentrations of 4-(n-pentylthio)-1,2-napthoquinone was investigated. The results of both studies are depicted in Fig. 21. The plots depict the survival of cultured MCF7 breast cancer cells (●) and adriamycin-resistant MCF7 breast cancer cells (MCF7ADR) (○).

### Example 18: 4-(2-N,N-dimethylamino-ethylamino)-1,2-napthoquinone Against MCF7 Cells and Adriamycin-Resistant MCF7 Cells

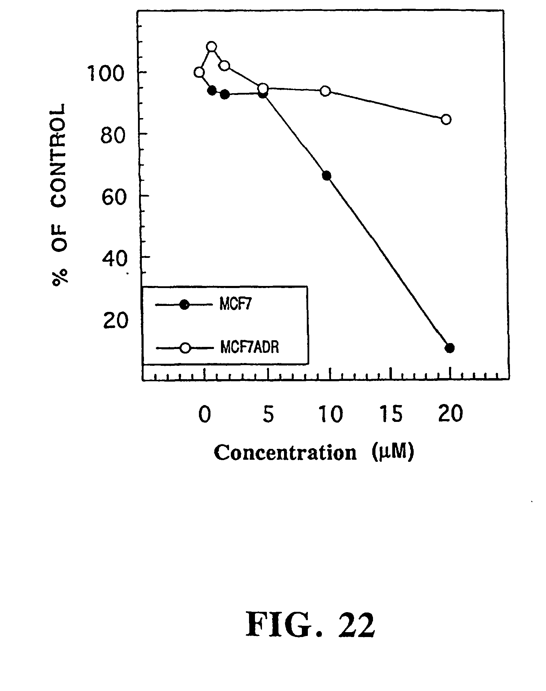

Following the standard protocol, the survival of cultured human breast cancer cells MCF7 and adriamycin-resistant MCF7 cells in the presence of increasing concentrations of 4-(2-N,N-dimethylamino-ethylamino)-1,2-napthoquinone was investigated. The results of both studies are depicted in Fig. 22. Here, the plots for the survival of cultured MCF7 breast cancer cells (●) and adriamycin-resistant MCF7 breast cancer cells (MCF7ADR) (○) are quite different. The compound tested had very little effect on the MCF7ADR cell line. But, while not as powerful as the compounds of Examples 6-17, at concentrations greater than about 10 µM, 4-(2-N,N-dimethylamino-ethylamino)-1,2-napthoquinoneeffectively inhibits the growth of MCF7 cells in culture.

### Example 19: 4-Pentyloxy-1,2-napthoquinone Against Atypical Multidrug-Resistant CEM/V-1 Cells

Following the standard protocol, the survival of cultured CEM leukemia cells and the survival of atypical multi-drug-resistant leukemia cells (CEM/V-1) in the presence of increasing concentrations of 4-pentyloxy-1,2-napthoquinone was investigated. The results of these two studies indicated an IC₅₀ of 0.25 µM against the CEM cells and an IC₅₀ of 0.35 µM against the multi-drug-resistant CEM/V-1 cells.

### Example 20: 4-Pentyloxy-1,2-napthoquinone Against Cell Lines which Over-Express MDR1: KB3-1 and KBH-1 Cells

Following the standard protocol, the survival of cultured KB3-1 and KBH-1 cell lines in the presence of increasing concentrations of 4-pentyloxy-1,2-napthoquinone was investigated. The results of these two studies indicated an IC₅₀ of 0.30 µM against the KB3-1 cells and an IC₅₀ of 0.15 µM against the KBH-1 cells.

### COMPARATIVE EXAMPLE:

### 4-Ethyloxy-1,2-napthoquinone Against A549 Cells and MCF7 Cells

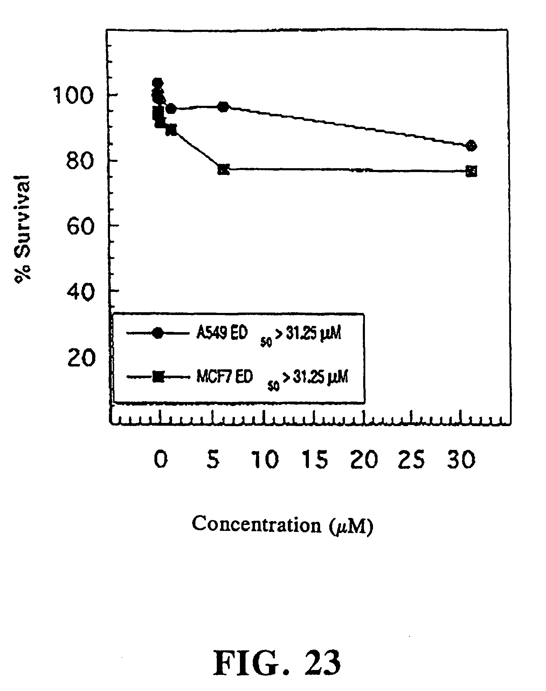

Following the standard protocol, the survival of cultured human lung cancer cells A549 and human breast cancer cells MCF7 in the presence of increasing concentrations of 4-ethyloxy-1,2-napthoquinone was investigated. The results of both studies are depicted in Fig. 23. The ED₅₀ of the ethyloxy derivative was shown to be 31.25 µM against both the A549 cells and the MCF7 cells.

It is understood that the invention is not confined to the particular chemical reactions, reagents, solvents, transformations, or cell lines herein illustrated and described, but embraces all such modified forms thereof as come within the scope of the following claims.

## Claims

1. A composition for use in the treatment of cancer in mammals **characterized in that** it includes an effective cancer cell-growth inhibiting amount of a compound of Formula I, II, or III: 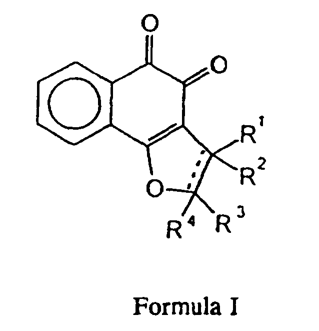 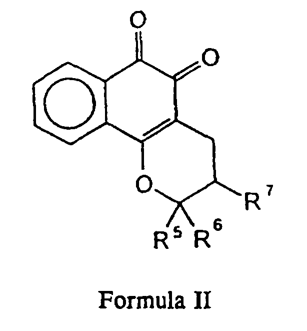 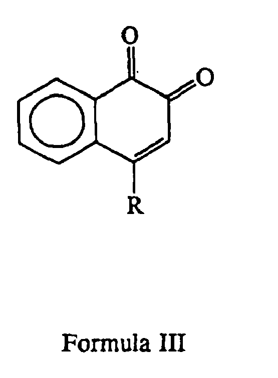 wherein R is chosen from C₃-C₈ linear, branched, or cyclic alkyloxy, C₅-C₈ linear or branched alkenyloxy, C₃-C₈ alkylthio, and C₄-C₈ linear or branched substituted alkyloxy, alkenyloxy, and alkylthio substituted with one or more substituents chosen from cycloalkyl, cycloalkenyl, cycloaryl, benzyl, and amino;
R¹ to R⁴ are each, independently, chosen from H, C₁-C₆ alkyl, C₁-C₆ alkenyl, C₁-C₆ alkoxy, C₁-C₆alkoxycarbonyl, -(CH₂)ₙ-aryl, -(CH₂)ₙ-heteroaryl, and -(CH₂-)ₙ₋phenyl, provided that at least one of R¹ to R⁴ is not hydrogen; or R¹ and R² combined are a single substituent chosen from the above group and R³ and R⁴ combined are a single substituent chosen from the above group, in which case --- is a double bond, and further provided that the combined R¹/R² and R³/R⁴ substituents are not simultaneously hydrogen;
R⁵ and R⁶ are each, independently, chosen from H, C₁-C₆ alkyl, C₁-C₆ alkenyl, C₁-C₆ alkoxy, C₁-C₆ alkoxycarbonyl, -(CH₂)ₙ-aryl, -(CH₂)ₙ-heteroaryl, and -(CH₂)ₙ₋phenyl;
R⁷ is chosen from C₁-C₆ acyloxy, -(CH₂)ₙ-amino, β-alanyloxy, malonyl, malonyloxy, -(CH₂)ₙ-aryl, -(CH₂)ₙ-heteroaryl, and -(CH₂)ₙ-phenyl; and
n is from 0 to 10, and salts thereof.

2. The composition according to claim 1, comprising pharmaceutically acceptable liquid or solid carrier.

3. The composition according to claim 1 or claim 2, wherein the compound is chosen from 3-(*O*-acetyl)-β-lapachone, 3-(β-alanyloxy)-β-lapachone, 3-malonyl-β-lapachone, dunnione, α-dunnione, 3-NOR-dunnione, 3,3-DINOR-dunnione, 3,3-DINOR-2,3-dehydrodunnione, 4-pentyloxy-1,2-naphthoquinone; 4-(2-methyl-propyloxy)-1,2-naphthoquinone; 4-(3-methyl-butyloxy)-1,2-naphthoquinone; 4-(isopropyloxy)-1,2-naphthoquinone; 4-(2-butenyloxy)-1,2-naphthoquinone; 4-benzyloxy-1,2-naphthoquinone; 4-cyclohexylmethyloxy-1,2-naphthoquinone; 4-(2-pentenyloxy)-1,2-naphthoquinone; 4-(n-heptyloxy)-1,2-naphthoquinone; 4-(n-pentylthio)-1,2-naphthoquinone; 4-(2-N,N-dimethylamino-ethylamino)-1,2-naphthoquinone, and pharmaceutically acceptable salts thereof

4. The composition according to any one of the preceding claims for use in the treatment of multiple-drug resistant cancer, adriamycin-resistant cancer, teniposide-resistant cancer, and vinblastine-resistant cancer.

5. A compound chosen from formula I, II or III: 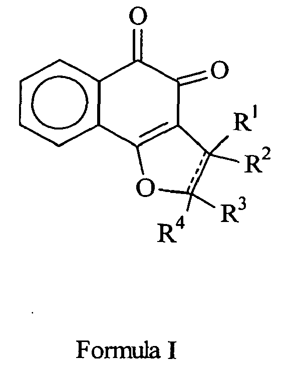 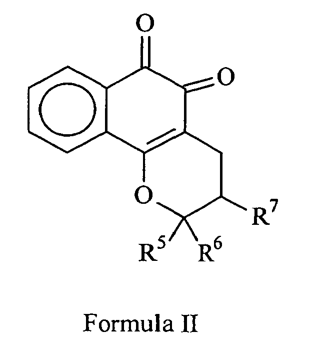 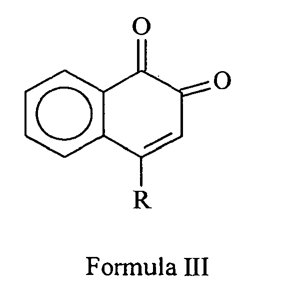 wherein R is chosen from cyclohexyl-C₁-C₈-alkyloxy, C₅-C₈ alkenyloxy, and C₃-C₈ linear or branched alkylthio;
R¹ to R⁴ are each, independently, chosen from C₁-C₆ alkyl, C₁-C₆ alkenyl, C₁₋₆ alkoxy, C₁-C₆ alkoxycarbonyl, (CH₂)ₙ-aryl, -(CH₂)ₙ-heteroaryl, and -(CH₂)ₙ-phenyl, or R¹ and R² combined are a single substituent chosen from the above group and R³ and R⁴ combined are a single substituent chosen from the above group, in which case --- is a double bond;
R⁵ and R⁶ are each, independently, chosen from H, C₁-C₆; alkyl, C₁-C₆ alkenyl, C₁-C₆ alkoxy, C₁-C₆ alkoxycarbonyl, -(CH₂)ₙ-aryl, -(CH₂)ₙ-heteroaryl, and -(CH₂)ₙ₋phenyl;
R⁷ is chosen from -(CH₂)ₙ-amino, β-alanyloxy, malonyl, malonyloxy, -(CH₂)ₙ₋aryl, -(CH₂)ₙ-heteroaryl, and -(CH₂)ₙ-phenyl; and
n is from 0 to 10, and salts thereof, except that the compound is not 3-(2'-aminoethyl)-β-lapachone.

6. A compound according to claim 5, chosen from 3-(β-alanyloxy)-β-lapachone and 3-malonyl-β-lapachone.

7. A compound according to claim 5 chosen from 4-(n-pentylthio)-1,2-napthoquinone, 4-(3-methyl-butyloxy)-1,2-napthoquinone, 4-cyclohexylmethyloxy-1,2-napthoquinone, 4-(2-pentenyloxy)-1,2-napthoquinone, and 4-(n-heptyloxy)-1,2-napthoquinone.

8. A method of synthesizing a compound of formula II 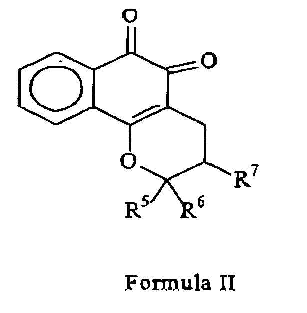 wherein R⁵ and R⁶ are each, independently chosen from H, C₁-C₆ alkyl, C₁-C₆ alkenyl, C₁-C₆ alkoxy, C₁-C₆ alkoxycarbonyl, -(CH₂)ₙ-aryl, -(CH₂)ₙ-heteroaryl,-(CH₂)ₙheterocycle, and -(CH₂)ₙ-phenyl; and
R⁷ is OH, C₁-C₆ alkyl, C₁-C₆ alkenyl, C₁-C₆ alkoxy, C₁-C₆ alkoxycarbonyl, C₁₋C₆ acyloxy, amino-C₂-C₆-acyloxy, malonyl, malonyloxy, β-alanyloxy, -(CH₂)ₙ₋amino, -(CH₂)ₙ-aryl, -(CH₂)ₙ-heteroaryl, -(CH₂)ₙ-heterocycle or -(CH₂)ₙ-phenyl; and wherein n is from 0 to 10;
comprising
(a) alkylating a Group IA metal salt of lawsone with an allyl halide of the formula : 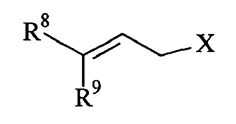 in the presence of M-I,
wherein R⁸ and R⁹ are each, independently, chosen from H, C₁-C₆ alkyl, C₁₋₆ alkenyl, C₁-C₆ alkoxy, C₁-C₆ alkoxycarbonyl, -(CH₂)ₙ-aryl, -(CH₂)ₙ-heteroaryl, - (CH₂)-heterocycle, and -(CH₂)ₙ-phenyl,
X is a halide, and
M is a Group IA metal;
to yield a mixture of C-alkylated and *O*-alkylated lawsone derivatives; and then
(b) cyclising the mixture of C-alkylated and *O*-alkylated lawsone derivatives to yield a tricyclic *ortho*-naphthoquinone.

9. A method according to claim 8, wherein in step (a), a lithium salt of lawsone is alkylated in the presence of lithium iodide.

10. A method according to claim 8 or 9, further comprising the step of separating the C-alkylated and O-alkylated lawsone derivatives synthesized in step (a) from one another prior to the cyclising in step (b).

11. A method according to any one of claims 8 to 10, wherein in step (b), the C-alkylated lawsone derivatives are cyclised by treatment with concentrated acid or by epoxidation followed by ring closure or by treatment with boron trifluoride.

12. A method according to any one of claims 8 to 11, further comprising rearranging the *O*-alkylated lawsone derivatives to yield C-alkylated derivatives and cyclising the same to yield a tricyclic *o*-naphthoquinone.

## Patentansprüche

1. Zusammensetzung zur Verwendung bei der Behandlung von Krebs bei Säugern, **dadurch gekennzeichnet, dass** sie eine das Wachstum von Krebszellen in wirksamer Weise hemmende Menge einer Verbindung der Formeln I, II oder III enthält: 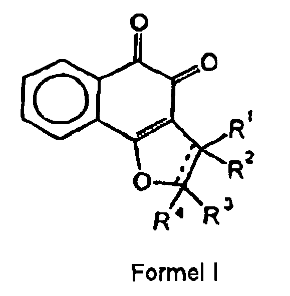 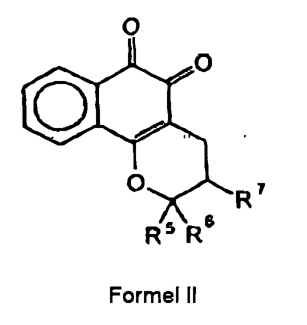 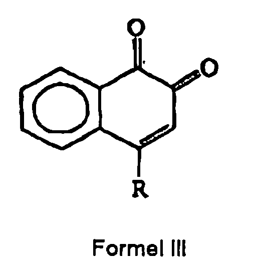 wobei R ausgewählt ist aus linearem, verzweigtem oder cyclischem C₃₋C₈-Alkyloxy, linearem oder verzweigtem C₅-C₈-Alkenyloxy, C₃-C₈-Alkylthio und linearem oder verzweigtem, substituiertem C₄-C₈-Alkyloxy, Alkenyloxy und Alkylthio, substituiert mit einem oder mehreren Substituenten, ausgewählt aus Cycloalkyl, Cycloalkenyl, Cycloaryl, Benzyl und Amino;
R¹ bis R⁴ jeweils unabhängig voneinander ausgewählt sind aus H, C₁-C₆-Alkyl, C₁-C₆-Alkenyl, C₁-C₆-Alkoxy, C₁-C₆-Alkoxycarbonyl, -(CH₂)ₙ₋Aryl, -(CH₂)ₙ-Heteroaryl und -(CH₂)ₙ-Phenyl, mit der Maßgabe, dass mindestens einer der Reste R¹ bis R⁴ nicht Wasserstoff bedeutet; oder R¹ und R² in Kombination einen einzigen Substituenten, der aus der vorstehenden Gruppe ausgewählt ist, darstellen und R³ und R⁴ in Kombination einen einzigen Substituenten, der aus der vorstehenden Gruppe ausgewählt ist, bedeuten, wobei in diesem Fall das Symbol --- eine Doppelbindung bedeutet, und mit der weiteren Maßgabe, dass die kombinierten R¹/R²- und R³/R⁴-Substituenten nicht gleichzeitig Wasserstoff bedeuten;
R⁵ und R⁶ jeweils unabhängig voneinander ausgewählt sind aus H, C₁-C₆-Alkyl, C₁-C₆-Alkenyl, C₁-C₆-Alkoxy, C₁-C₆-Alkoxycarbonyl, -(CH₂)ₙ₋Aryl, -(CH₂)ₙ-Heteroaryl und -(CH₂)ₙ-Phenyl;
R⁷ ausgewählt ist aus C₁-C₆-Acyloxy, -(CH₂)ₙ-Amino, β-Alanyloxy, Malonyl, Malonyloxy, -(CH₂)ₙ-Aryl, -(CH₂)ₙ-Heteroaryl und -(CH₂)ₙ-Phenyl; und
n einen Wert von 0 bis 10 hat,
sowie Salze davon.

2. Zusammensetzung nach Anspruch 1, umfassend einen pharmazeutisch verträglichen flüssigen oder festen Träger .

3. Zusammensetzung nach Anspruch 1 oder 2, wobei die Verbindung ausgewählt ist aus 3-(O-Acetyl)-β-lapachon, 3-(β-Alanyloxy)-β-lapachon, 3-Malonyl-β-lapachon, Dunnion, α-Dunnion, 3-NOR-dunnion, 3,3-DINORdunnion, 3,3-DINOR-2,3-dehydrodunnion, 4-Pentyloxy-1,2-naphthochinon, 4-(2-methylpropyloxy)-1,2-naphthochinon, 4-(3-Methylbutyloxy)-1,2-naphthochinon, 4-(Isopropyloxy)-1,2-naphthochinon, 4-(2-Butenyloxy),-1,2-naphthochinon, 4-Benyzloxy-1,2-naphthochinon, 4-Cyclohexylmethyloxy-1,2-naphthochinon, 4-(2-Pentenyloxy)-1,2-naphthochinon, 4-(n-Heptyloxy)-1,2-naphthochinon, 4-(n-Pentylthio)-1,2-naphthochinon, 4-(2-N,N-Dimethylaminoethylamino)-1,2-naphthochinon, sowie pharmazeutisch verträgliche Salze davon.

4. Zusammensetzung nach einem der vorstehenden Ansprüche zur Verwendung bei der Behandlung eines gegen mehrere Arzneistoffe resistenten Krebses, eines Adriamycin-resistenten Krebses, eines Teniposid-resistenten Krebses und eines Vinblastin-resistenten Krebses.

5. Verbindung, ausgewählt aus den Formeln I, II oder III 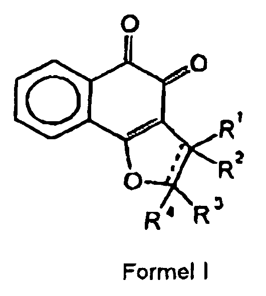 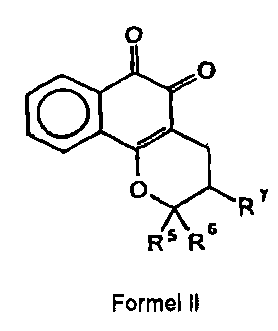 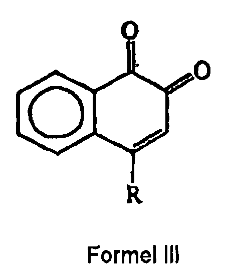 wobei R ausgewählt ist aus Cyclohexyl-C₁-C₈-Alkyloxy, C₅-C₈₋Alkenyloxy und linearem oder verzweigtem C₃-C₈-Alkylthio;
R¹ bis R⁴ jeweils unabhängig voneinander ausgewählt sind aus C₁-C₆₋Alkyl, C₁-C₆-Alkenyl, C₁-C₆-Alkoxy, C₁-C₆-Alkoxycarbonyl, -(CH2)ₙ-Aryl, -(CH₂)ₙ-Heteroaryl und -(CH₂)ₙ-Phenyl oder R¹ und R² in Kombination einen einzigen Substituenten, der aus der vorstehenden Gruppe ausgewählt ist, bedeuten und R³ und R⁴ in Kombination einen einzigen Substituenten, der aus der vorstehenden Gruppe ausgewählt ist, bedeuten, wobei in diesem Fall das Symbol --- eine Doppelbindung bedeutet;
R⁵ und R⁶ jeweils unabhängig voneinander ausgewählt sind aus H, C₁-C₆-Alkyl, C₁-C₆-Alkenyl, C₁-C₆-Alkoxy, C₁-C₆-Alkoxycarbonyl, -(CH₂)ₙ₋Aryl, -(CH₂)ₙ-Heteroaryl und -(CH₂)ₙ-Phenyl;
R⁷ ausgewählt ist aus -(CH₂)ₙ-Amino, β-Alanyloxy, Malonyl, Malonyloxy, -(CH₂)ₙ-Aryl, -(CH₂)ₙ-Heteroaryl und -(CH₂)ₙ-Phenyl; und
n einen Wert von 0 bis 10 hat sowie Salze davon, mit der Ausnahme, dass es sich bei der Verbindung nicht um 3-(2'-Aminoethyl)-β-lapachon handelt.

6. Verbindung nach Anspruch 5, ausgewählt aus 3-(β-Alanyloxy)-β-lapachon und 3-Malonyl-β-lapachon.

7. Verbindung nach Anspruch 5, ausgewählt aus 4-(n-Pentylthio)-1,2-naphthochinon, 4-(3-Methylbutyloxy)-1,2-naphthochinon, 4-Cyclohexylmethyloxy-1,2-naphthochinon, 4-(2-Pentenyloxy)-1,2-naphthochinon und 9-(n-Heptyloxy)-1,2-naphthochinon.

8. Verfahren zur Herstellung einer Verbindung der Formel II 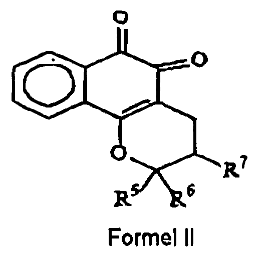 wobei R⁵ und R⁶ jeweils unabhängig voneinander ausgewählt sind aus H, C₁-C₆-Alkyl, C₁-C₆-Alkenyl, C₁-C₆-Alkoxy, C₁-C₆-Alkoxycarbonyl, -(CH₂)ₙ-Aryl, -(CH₂)ₙ-Heteroaryl, -(CH₂)ₙ-Heterocyclus und -(CH₂)ₙ₋Phenyl; und
R⁷ OH, C₁-C₆-Alkyl, C₁-C₆-Alkenyl, C₁-C₆-Alkoxy, C₁-C₆₋Alkoxycarbonyl, C₁-C₆-Acyloxy, Amino-C₂-C₆-acyloxy, Malonyl, Malonyloxy, β-Alanyloxy, -(CH₂)ₙ-Amino, -(CH₂)ₙ-Aryl, -(CH₂)ₙ-Heteroaryl, -(CH₂)ₙ₋Heterocyclus oder -(CH₂)ₙ-Phenyl bedeutet; und
wobei n einen Wert von 0 bis 10 hat;
umfassend
(a) das Alkylieren eines Gruppe IA-Metallsalzes von Lawson mit einem Allylhalogenid der Formel 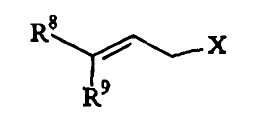 in Gegenwart von M-I,
wobei R⁸ und R⁹ jeweils unabhängig voneinander ausgewählt sind aus H, C₁-C₆-Alkyl, C₁-C₆-Alkenyl, C₁-C₆-Alkoxy, C₁-C₆-Alkoxycarbonyl, -(CH₂)ₙ-Aryl, -(CH₂)ₙ-Heteroaryl, -(CH₂)ₙ-Heterocyclus und -(CH₂)ₙ-Phenyl,
X ein Halogenid bedeutet und
M ein Metall der Gruppe IA bedeutet;
unter Bildung eines Gemisches von C-alkylierten und O-alkylierten Lawson-Derivaten; und anschließend
(b) das Cyclisieren des Gemisches der C-alkylierten und O-alkylierten Lawson-Derivate unter Bildung eines tricyclischen ortho-Naphthochinons.

9. Verfahren nach Anspruch 8, wobei in Stufe (a) ein Lithiumsalz von Lawson in Gegenwart von Lithiumiodid alkyliert wird.

10. Verfahren nach Anspruch 8 oder 9, ferner umfassend die Stufe des Trennens der C-alkylierten und O-alkylierten Lawson-Derivate, die in Stufe (a) hergestellt worden sind, voneinander vor der Cyclisierung in Stufe (b).

11. Verfahren nach einem der Ansprüche 8 bis 10, wobei in Stufe (b) die C-alkylierten Lawson-Derivate durch Behandlung mit konzentrierter Säure oder durch Epoxidation unter anschließendem Ringschluss oder Behandlung mit Bortrifluorid cyclisiert werden.

12. Verfahren nach einem der Ansprüche 6 bis 11, ferner umfassend das Umlagern der O-alkylierten Lawson-Derivate unter Bildung von C-alkylierten Derivaten und Cyclisieren dieser Produkte unter Bildung eines tricyclischen o-Naphthochinons,

## Revendications

1. Composition utilisable dans le traitement d'un cancer chez des mammifères, **caractérisée en ce qu'**elle contient, en une quantité à effet d'inhibition de la prolifération de cellules cancéreuses, un composé de formule I, II ou III : 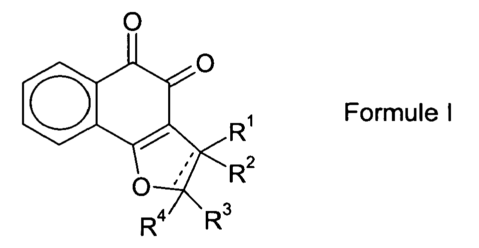 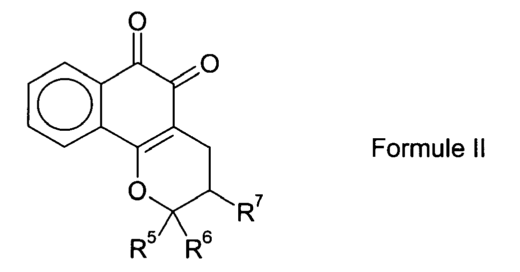 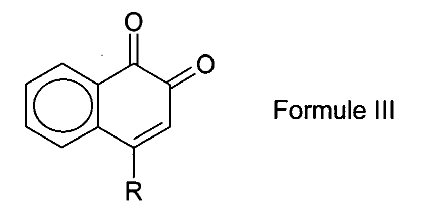 dans lesquelles
- R représente une entité choisie parmi les groupes alcoxy en C₃₋₈ à chaîne linéaire, ramifiée ou cyclique, les groupes alcényloxy en C₅₋₈ à chaîne linéaire ou ramifiée, les groupes alkylthio en C₃₋₈, et les groupes alcoxy, alcényloxy ou alkylthio en C₄₋₈ à chaîne linéaire ou ramifiée et portant un ou plusieurs substituants choisis parmi les groupes cycloalkyle, cycloalcényle, aryle, benzyle et amino ;
- les symboles R¹ à R⁴ représentent chacun, indépendamment, une entité choisie parmi un atome d'hydrogène et les groupes alkyle en C₁₋₆, alcényle en C₁₋₆, alcoxy en C₁₋₆, (alcoxy en C₁₋₆)carbonyle, -(CH₂)ₙ-aryle, -(CH₂)ₙ-hétéroaryle et -(CH₂)ₙ-phényle, sous réserve qu'au moins l'un des symboles R¹ à R⁴ ne représente pas un atome d'hydrogène, ou bien R¹ et R² représentent conjointement un substituant unique choisi parmi ceux mentionnés ci-dessus et R³ et R⁴ représentent conjointement un substituant unique choisi parmi ceux mentionnés ci-dessus, auquel cas le trait en pointillés représente une liaison formant une double liaison, sous réserve que les paires de symboles R¹/R² et R³/R⁴ ne représentent pas simultanément chacune un atome d'hydrogène ;
- les symboles R⁵ et R⁶ représentent chacun, indépendamment, une entité choisie parmi un atome d'hydrogène et les groupes alkyle en C₁₋₆, alcényle en C₁₋₆, alcoxy en C₁₋₆, (alcoxy en C₁-₆)carbonyle, -(CH₂)ₙ-aryle,
- (CH₂)ₙ-hétéroaryle et -(CH₂)ₙ-phényle ;
- le symbole R⁷ représente une entité choisie parmi les groupes acyloxy en C₁-₆, -(CH₂)ₙ-amino, β-alanyloxy, malonyle, malonyloxy, -(CH₂)ₙ-aryle, -(CH₂)ₙ-hétéroaryle et -(CH₂)ₙ-phényle ;
- et n vaut de 0 à 10 ;
ou un sel d'un tel composé.

2. Composition conforme à la revendication 1, comprenant un véhicule liquide ou solide, pharmacologiquement admissible.

3. Composition conforme à la revendication 1 ou 2, dans laquelle le composé est choisi parmi les suivants : 3-(O-acétyl)-β-lapachone, 3-(β-alanyloxy)-β-lapachone, 3-malonyl-β-lapachone, dunnione, α-dunnione, 3-nor-dunnione, 3,3-dinor-dunnionne, 3,3-dinor-2,3-déhydrodunnione, 4-pentyloxy-1,2-naphtoquinone, 4-(2-méthyl-propyloxy)-1,2-naphtoquinone, 4-(3-méthyl-butyloxy)-1,2-naphtoquinone, 4-(isopropyloxy)-1,2-naphtoquinone, 4-(2-butényloxy)-1,2-naphtoquinone, 4-benzyloxy-1,2-naphtoquinone, 4-cyclohexylméthyloxy-1,2-naphtoquinone, 4-(2-pentényloxy)-1,2-naphtoquinone, 4-n-heptyloxy-1,2-naphtoquinone, 4-n-pentylthio-1,2-naphtoquinone et 4-[2-(N,N-diméthylamino)éthylamino]-1,2-naphtoquinone, ainsi que parmi leurs sels pharmacologiquement admissibles.

4. Composition conforme à l'une des revendications précédentes, utilisable pour le traitement d'un cancer résistant à une polychimiothérapie, d'un cancer résistant à l'adriamycine, d'un cancer résistant au téniposide, ou d'un cancer résistant à la vinblastine.

5. Composé choisi parmi ceux de formule I, II ou III : 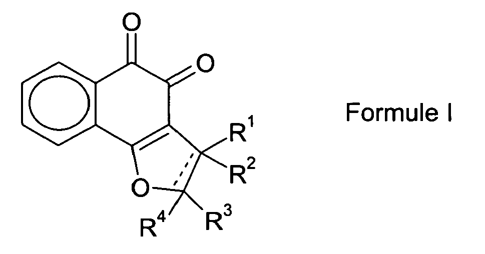 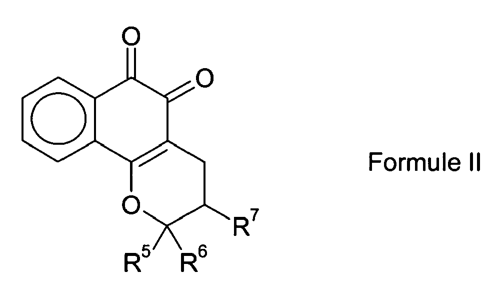 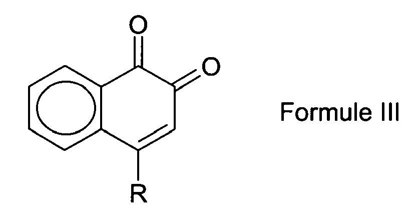 dans lesquelles
- R représente une entité choisie parmi les groupes cyclohexyl-(alcoxy en C₁₋₈), les groupes alcényloxy en C₅₋₈, et les groupes alkylthio en C₃₋₈ à chaîne linéaire ou ramifiée ;
- les symboles R¹ à R⁴ représentent chacun, indépendamment, une entité choisie parmi les groupes alkyle en C₁₋₆, alcényle en C₁₋₆, alcoxy en C₁₋₆, (alcoxy en C₁₋₆)carbonyle, -(CH₂)ₙ-aryle, -(CH₂)ₙ-hétéroaryle et -(CH₂)ₙ-phényle, ou bien R¹ et R² représentent conjointement un substituant unique choisi parmi ceux mentionnés ci-dessus et R³ et R⁴ représentent conjointement un substituant unique choisi parmi ceux mentionnés ci-dessus, auquel cas le trait en pointillés représente une liaison formant une double liaison ;
- les symboles R⁵ et R⁶ représentent chacun, indépendamment, une entité choisie parmi un atome d'hydrogène et les groupes alkyle en C₁₋₆, alcényle en C₁₋₆, alcoxy en C₁₋₆, (alcoxy en C₁₋₆)carbonyle, -(CH₂)ₙ-aryle, -(CH₂)ₙ-hétéroaryle et -(CH₂)ₙ-phényle ;
- le symbole R⁷ représente une entité choisie parmi les groupes -(CH₂)ₙ-amino, β-alanyloxy, malonyle, malonyloxy, -(CH₂)ₙ-aryle, -(CH₂)ₙ-hétéroaryle et -(CH₂)ₙ-phényle ;
- et n vaut de 0 à 10 ;
et les sels de tels composés, sous réserve qu'il ne s'agisse pas de la 3-(2'-aminoéthyl)-β-lapachone.

6. Composé conforme à la revendication 5, choisi parmi la 3-(β-alanyloxy)-β-lapachone et la 3-malonyl-β-lapachone.

7. Composé conforme à la revendication 5, choisi parmi les 4-n-pentylthio-1,2-naphtoquinone, 4-(3-méthyl-butyloxy)-1,2-naphtoquinone, 4-cyclohexylméthyloxy-1,2-naphtoquinone, 4-(2-pentényloxy)-1,2-naphtoquinone et 4-n-heptyloxy-1,2-naphtoquinone.

8. Procédé de synthèse d'un composé de formule II : 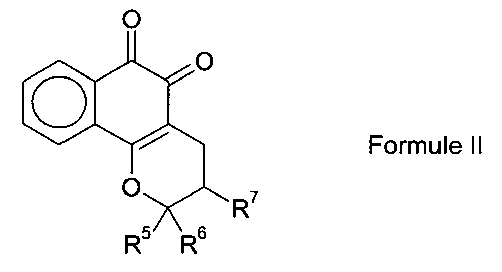 dans laquelle
- les symboles R⁵ et R⁶ représentent chacun, indépendamment, une entité choisie parmi un atome d'hydrogène et les groupes alkyle en C₁₋₆, alcényle en C₁₋₆, alcoxy en C₁₋₆, (alcoxy en C₁₋₆)carbonyle, -(CH₂)ₙ-aryle, -(CH₂)ₙ-hétéroaryle, -(CH₂)ₙ-hétérocyclyle et -(CH₂)ₙ-phényle ;
- le symbole R⁷ représente une entité choisie parmi les groupes hydroxyle, alkyle en C₁₋₆, alcényle en C₁₋₆, alcoxy en C₁₋₆, (alcoxy en C₁₋₆)carbony-le, amino-acyloxy en C₂₋₆, malonyle, malonyloxy, β-alanyloxy, -(CH₂)ₙ-amino, -(CH₂)ₙ-aryle, -(CH₂)ₙ-hétéroaryle, -(CH₂)ₙ-hétérocyclyle et -(CH₂)ₙ-phényle ;
- et n vaut de 0 à 10;
lequel procédé comporte :
a) le fait d'alkyler un sel de lawsone et d'un métal du Groupe IA, avec un halogénure d'allyle de formule 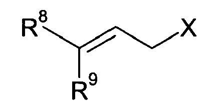 et en présence d'un iodure de formule M-I,
formules dans lesquelles
- R⁸ et R⁹ représentent chacun, indépendamment, une entité choisie parmi un atome d'hydrogène et les groupes alkyle en C₁-₆, alcényle en C₁₋₆, alcoxy en C₁₋₆, (alcoxy en C₁₋₆)carbonyle, -(CH₂)ₙ-aryle, -(CH₂)ₙ-hétéroaryle, -(CH₂)ₙ-hétérocyclyle et -(CH₂)ₙ-phényle,
- X représente un atome d'halogène,
- et M représente un atome d'un métal du Groupe IA,
de manière à ce que cela donne un mélange de dérivé C-alkylé et de dérivé O-alkylé de lawsone,
b) et le fait de soumettre ce mélange de dérivés C-alkylé et O-alkylé de lawsone à une réaction de cyclisation, de manière à ce que cela donne une ortho-naphtoquinone tricyclique.

9. Procédé conforme à la revendication 8, dans lequel, dans l'étape (a), on effectue l'alkylation d'un sel de lithium de lawsone, en présence de iodure de lithium.

10. Procédé conforme à la revendication 8 ou 9, qui comporte en outre une étape où l'on sépare l'un de l'autre les dérivés C-alkylé et O-alkylé de lawsone synthétisés au cours de l'étape (a), avant d'effectuer la cyclisation de l'étape (b).

11. Procédé conforme à l'une des revendications 8 à 10, dans lequel, dans l'étape (b), on effectue la cyclisation du dérivé C-alkylé de lawsone par traitement avec un acide concentré, ou par époxydation suivie d'une réaction de fermeture de cycle par traitement avec du trifluorure de bore.

12. Procédé conforme à l'une des revendications 8 à 11, qui comporte en outre une étape de réarrangement du dérivé O-alkylé de lawsone en dérivé C-alkylé, puis la cyclisation de ce dernier en une ortho-naphtoquinone tricyclique.
